# EUROPEAN PATENT SPECIFICATION

(11) **EP 2 605 790 B1**
(45) Date of publication and mention of the grant of the patent: **08.07.2020**
(21) Application number: 11818641.0
(22) Date of filing: 16.08.2011
(51) Int. Cl.: A61K 47/64, A61K 38/48, A61K 38/55, A61K 9/00, C07K 16/18, A61P 25/00

(54) **INTRANASAL DELIVERY OF CELL PERMEANT THERAPEUTICS**
INTRANASALE VERABREICHUNG ZELLDURCHLÄSSIGER THERAPIEMITTEL
ADMINISTRATION INTRANASALE D'AGENTS THÉRAPEUTIQUES AUGMENTANT LA PERMÉABILITÉ CELLULAIRE

(30) Priority: 16.08.2010 US 374113 P
(43) Date of publication of application: 26.06.2013
(73) Proprietor: The Trustees of Columbia University in the City of New York, New York, NY 10027 (US)
(72) Inventor: TROY, Carol, M., Hastings-on-Hudson, NY 10706 (US); AKPAN, Nsikan, New York, NY 10027 (US); SALVESEN, Guy, Encinitas, CA 92024 (US); SNIPES, Scott, La Jolla, CA 92037 (US)
(74) Representative: Barker Brettell LLP
(86) International application number: PCT/US2011/047858
(87) International publication number: WO 2012/024260

(56) References cited:
- WO-A1-91/07947
- US-A1- 2002 082 215
- US-A1- 2003 166 192
- US-A1- 2009 042 783
- US-A1- 2009 118 180
- US-A1- 2009 280 058
- US-B2- 7 439 063
- SERRRANO-SAIZ E ET AL: "Inhibition of caspase-9 activity in a rat model of ischemia provides substantial neuroprotection", SOCIETY FOR NEUROSCIENCE ABSTRACT VIEWER AND ITINERARY PLANNER, vol. 38, 2008, XP0009180444, & 38TH ANNUAL MEETING OF THE SOCIETY-FOR-NEUROSCIENCE; WASHINGTON, DC, USA; NOVEMBER 15 -19, 2008
- FAN Y F ET AL: "Apoptosis inhibition in ischemic brain by intraperitoneal PTD-BIR3-RING (XIAP)", NEUROCHEMISTRY INTERNATIONAL, PERGAMON PRESS, OXFORD, GB, vol. 48, no. 1, 1 January 2006 (2006-01-01), pages 50-59, XP027957891, ISSN: 0197-0186 [retrieved on 2006-01-01]
- KHAFAGY E S ET AL: "Efficiency of cell-penetrating peptides on the nasal and intestinal absorption of therapeutic peptides and proteins", INTERNATIONAL JOURNAL OF PHARMACEUTICS, ELSEVIER BV, NL, vol. 381, no. 1, 20 October 2009 (2009-10-20), pages 49-55, XP026642142, ISSN: 0378-5173, DOI: 10.1016/J.IJPHARM.2009.07.022 [retrieved on 2009-07-29]
- AKPAN NSIKAN ET AL: "Intranasal Delivery of Caspase-9 Inhibitor Reduces Caspase-6-Dependent Axon/Neuron Loss and Improves Neurological Function after Stroke", JOURNAL OF NEUROSCIENCE, vol. 31, no. 24, June 2011 (2011-06), pages 8894-8904, XP002730585, ISSN: 0270-6474
- DRUILHE ET AL.: 'Regulation of IL-1 beta generation by Pseudo-ICE and ICEBERG, two dominant negative caspase recruitment domain proteins.' CELL DEATH AND DIFFERENTIATION vol. 8, 2001, pages 649 - 657, XP001079961
- KAMEI N ET AL: "Permeation characteristics of oligoarginine through intestinal epithelium and its usefulness for intestinal peptide drug delivery", JOURNAL OF CONTROLLED RELEASE, ELSEVIER, AMSTERDAM, NL, vol. 131, no. 2, 21 October 2008 (2008-10-21), pages 94-99, XP025478794, ISSN: 0168-3659, DOI: 10.1016/J.JCONREL.2008.07.016 [retrieved on 2008-07-18]
- KLEMENTIEV B ET AL: "A neural cell adhesion molecule-derived peptide reduces neuropathological signs and cognitive impairment induced by Asz25-35", NEUROSCIENCE, NEW YORK, NY, US, vol. 145, no. 1, 2 March 2007 (2007-03-02) , pages 209-224, XP026865478, ISSN: 0306-4522 [retrieved on 2007-02-13]

## Description

### GRANT INFORMATION

This invention was made with government support under grant numbers NS43089 and NS37878 awarded by National Institutes of Health. The government has certain rights in the invention

### PRIORITY

This application claims the benefit of the filing date of United States provisional application serial no. 61/374,113, filed August 16, 2010.

### 1. INTRODUCTION

The present invention relates to compositions for use in the treatment of ischemic injury in the central nervous system ("CNS"). Also, disclosed herein are compositions and methods useful for extending the therapeutic window associated with CNS ischemic injury.

### 2. BACKGROUND OF THE INVENTION

Stroke is the third leading cause of death and the leading cause of motor disability in the industrialized world. In ischemic stroke, which accounts for 85% of all stroke cases, thrombosis or embolism leads to an occlusion of a major artery that supplies the brain with oxygen, and depletion of oxygen results in tissue injury. The injured territory downstream from the occlusion is comprised of an ischemic core and its surrounding penumbra. The ischemic core is the territory where perfusion decreased below the threshold for viability, and where the cells are both electrically silent and irreversibly injured. Injury to the core occurs primarily via necrosis, however, there is recent evidence arguing that apoptosis may also occur in the core. (Yuan, Apoptosis 14 (4), 469-477 (2009)). In contrast, the area defined as the penumbra continues to receive blood and nutrients, although at a reduced capacity, and these cells could potentially remain viable. When cell death occurs in the penumbra, it is thought to be due to apoptosis. (Ribe, et al., Biochem J 415 (2), 165-182 (2008)). With timely reperfusion, either spontaneous or therapeutic, this territory may be salvaged. However, restoration of blood flow can also induce 'reperfusion injury', which exacerbates inflammation, excitotoxicity, and apoptotic cell injury. (Ribe, et al, Biochem J 415 (2), 165-182 (2008)). In humans, apoptotic markers, including cleaved caspases, can be observed in the peri-infarct region from 24hrs to 26 days following a stroke and diffusion tensor imaging reveals extensive loss of axonal tracts in the stroke penumbra. (Broughton, et al., Stroke 40 (5), e331-339 (2009); Mitsios, et al., Cell Biochem Biophys 47 (1), 73-86 (2007); Lie, et al., Stroke 35 (1), 86-92 (2004); Thomalla, et al., Neuroimage 22 (4), 1767-1774 (2004)).

Axon degeneration, such as that identified in the stroke penumbra, is generally characterized by axonal swelling, poor or halted axon transport, and fragmentation. This degeneration is not simply a marker of neuron death, but also plays an active role in provoking/ promoting neuronal death. (Ferri, et al., Curr Biol 13 (8), 669-673 (2003); Fischer, et al., Exp Neurol 185 (2), 232-240 (2004); Stokin, et al., Science 307 (5713), 1282-1288 (2005); Li, et al., J Neurosci 21 (21), 8473-8481 (2001); Coleman, Nat Rev Neurosci 6 (11), 889-898 (2005)). For example, a pathologic role has been reported for axon degeneration in Huntington's disease and motor neuron diseases, such as ALS, and it is also a hallmark of acute neurological disease, including stroke and traumatic brain injury. (Ferri, et al.,. Curr Biol 13 (8), 669-673 (2003); Fischer, et al., Exp Neurol 185 (2), 232-240 (2004); Stokin, et al., Science 307 (5713), 1282-1288 (2005); Li, et al., J Neurosci 21 (21), 8473-8481 (2001)). Optic nerve cultures under anoxic conditions exhibit fragmenting of the axonal cytoskeleton and deficits in fast axonal transport. (Waxman, et al., Brain Res 574 (1-2), 105-119 (1992)). Following transient middle cerebral artery occlusion (tMCAo) in rodents, there is selective damage of microtubules, neurofilaments, and associated proteins in the axon, including tau. (Dewar, et al., Brain Res 684 (1), 70-78 (1995); Dewar, et al., Acta Neuropathol 93 (1), 71-77 (1997)). Additionally, the number of spines and axon terminals decreases around 12-24 hours post-reperfusion (hpr) following gerbil tMCAo. (Ito, et al., Stroke 37 (8), 2134-2139 (2006)). Furthermore, WldS (slow wallerian degeneration) mutant mice display marked resistance to axon degeneration, and these mice are protected from cerebral ischemia. (Gillingwater, et al., J Cereb Blood Flow Metab 24 (1), 62-66 (2004)). Therefore, preventing initial axon destruction can limit subsequent functional neurologic deficits following stroke.

As noted above, the members of the caspase family of proteins (including caspases -1, -2, -3, -4, -5, -6, -7, -8, -9, 10, -11, -12, and -14) have been identified as apoptotic molecules that become activated following ischemic injury. It is also known that inhibition of Caspase-9 activity in a rat model of ischemia provides neuroprotection (Serrrano-Saiz et al.. Society for Neuroscience Abstract Viewer and itinerary planner, Vol. 38 (2008) - 38th Annual meeting of the society for neuroscience; Washington, DC, US. 15 - 19 November 2008) Also, there are a number of putative mechanisms in connection with caspase-9's role in inducing apoptosis after ischemic injury. In one mechanism, reactive oxygen species are first generated by hypoxia, which results in DNA damage and the activation of p53. (Niizuma, et al., J Neurochem 109 Suppl 1, 133-138 (2009)). During apoptosis, activated p53 translocates to the mitochondrial outer membrane where it recruits Bcl-2 associated X protein (Bax) and other proapoptotic proteins. This recruitment leads to permeabilization of the outer mitochondrial membrane and releases cytochrome c into the cytosol, which leads to the activation of caspase-9. Alternatively, activation of caspase-9 and the resulting apoptosis activation in ischemia could be receptor mediated. Both p75-neurotrophin receptor (p75NTR) and death receptor 6 (DR6) stimulation result in caspase-6 activation, and with DR6, axon degeneration. (Troy, et al., J Biol Chem 277 (37), 34295-34302 (2002); Nikolaev, et al., Nature 457 (7232), 981-989 (2009)). One of the many downstream targets of p75NTR is p53. One of the interacting partners of DR6 is the tumor necrosis factor receptor type 1-associated death domain (TRADD), which binding to signal transducer TRAF2 and activates NF-kappaB. In relation to cell death function, NF-kappaB has both pro-apoptotic and anti-apoptotic function, but persistent activation of NF-kappaB in stroke is thought to be associated with driving a proapoptotic fate. (Ridder, et al.. Neuroscience 158 (3), 995-1006 (2009)). NF-kappaB regulates Bcl-2 family members (Bim, Bid, Bax, Bak) to effect mitochondrial membrane stability, cytochrome c release, and subsequently caspase-9 activation leading to apoptosis. (Ridder, et al., Neuroscience 158 (3), 995-1006 (2009)).

Similarly, caspase-6 has been implicated in neuronal death in multiple neurodegenerative diseases. Initial analysis of proteolytic substrates of caspase-6 in vitro identified lamins and poly ADP ribose polymerase (PARP) as targets. (Orth, et al., A. J Biol Chem 271 (28), 16443-16446 (1996); Takahashi, et al., Proc Natl Acad Sci U S A 93 (16), 8395-8400 (1996)). Since these targets are also common to caspase-3, these observations led to the common assumption that caspase-6 and caspase-3 played redundant roles in mediating nuclear degradation during neuronal apoptosis. However, recent evidence shows caspase-6 can specifically mediate the cleavage of non-nuclear targets. (Klaiman, et al., Mol Cell Proteomics 7 (8), 1541-1555 (2008); Graham, et al., Cell 125 (6), 1179-1191 (2006); Guo, et al., Am J Pathol 165 (2), 523-531 (2004)). For example, in Huntington's disease, cleavage of mutant huntingtin by caspase-6, and not caspase-3, is necessary for neurodegeneration. (Graham, et al., Cell 125 (6), 1179-1191 (2006)). In Alzheimer's disease (AD), neuropil threads contain caspase-6 cleaved tau and tubulin, suggesting a function for caspase-6 in axonal degeneration in AD. (Klaiman, et al., Mol Cell Proteomics 7 (8), 1541-1555 (2008); Guo, et al., Am J Pathol 165 (2), 523-531 (2004)). Furthermore, caspase-6 mediates axon degeneration in sensory neurons following nerve growth factor (NGF) deprivation in a caspase-3 independent manner. (Nikolaev, et al., Nature 457 (7232), 981-989 (2009)).

Although certain proteins involved with apoptosis, including the above-described caspases, are potential targets for therapeutic intervention in ischemic injury, current pharmacologic therapies are instead focused on thrombolytics. Thrombolytic therapeutics function to restore blood flow to the site of an ischemic injury by breaking down the fibrin fibers that have associated to form a blood clot, where the blood clot is itself the cause of the ischemic injury. Examples of thrombolytics currently marketed for use in treating ischemic injury include streptokinase, tissue plasminogen activator (tPA), and urokinase. Unfortunately, the use of thrombolytics is significantly restricted, not only due on their limited therapeutic window, but also in light of the serious side effects associated with their use.

To determine the appropriate therapeutic window in which thrombolytics can be administered, a clinical trial conducted by the National Institute of Neurologic Disorder and Stroke, the NINDS recombinant tPA Stroke Trial. (Marler et al., N Engl J Med 333 (24), 1581-1588 (1995)). This trial concentrated on the effect of intravenous recombinant tPA treatment within three hours after the onset of the symptoms. Due to the observed positive effects of this treatment on the viability of patients, recombinant tPA treatment within the limited time frame of three hours post-onset of the ischemic injury was recommended. However, even within this narrow window, the authors did find a higher risk for intracerebral hemorrhage ("ICH"). Additional studies have attempted to determine whether the therapeutic window could be enlarged, however, the general use of recombinant tPA within 6 hours after the onset of stroke was ultimately not recommended as administration during that enlarged window increased the risk of ICH. (Lewandowski and Barsan, Annals of Emergency Medicine 37 (2) S. 202 ff. (2001)).

In addition to the limited window for administering thrombolytics, use of such therapeutics is associated with significant deleterious side effects. For example, therapy with streptokinase has severe disadvantages since it is a bacterial protease and therefore can provoke allergic reactions in the body. In addition, if a patient has previously experienced a streptococci infection, the patient may exhibit streptokinase resistance making the therapy even more problematic. Furthermore, clinical trials in Europe (Multicenter Acute Stroke Trial of Europe (MAST-E), Multicenter Acute Stroke Trial of Italy (MAST-I)) and Australia (Australian Streptokinase Trial (AST)) indicated an increased mortality risk and a higher risk of ICH after treatment with streptokinase and in certain instances these trials had to be terminated early. (Jaillard et al., Stroke 30, 1326-1332 (1999); Motto et al., Stroke 30 (4), 761-4 (1999); Yasaka et al., Neurology 50 (3), 626-32 (1998)). Furthermore, although recombinant tPA was ultimately approved by FDA for use in connection with ischemic injury, this approval was granted despite its known neurotoxic side effects and its negative effect on mortality.

In light of the foregoing, identification of the specific duration of activity of specific apoptotic targets, such as cleaved caspases, would not only be advantageous to further define their utility as therapeutic targets for inhibiting apoptotic activity associated with ischemic injury, but would also allow for an extension of the current therapeutic window for stroke.

### 3. SUMMARY OF THE INVENTION

There is provided a compound for use in the treatment of ischemic injury in the central nervous system, wherein the compound is administered intranasally and comprises a caspase inhibitor covalently conjugated to a cell-penetrating peptide, wherein said caspase inhibitor is selected from the group consisting of: (i) a caspase-6 dominant negative (C6DN), (ii) XBIR3, and (iii) a peptide that is capable of apoptotic target inhibition and has at least 70% homology with either a C6DN or a XBIR3; and said cell-penetrating peptide is selected from the group consisting of penetratin-1, transportan, pIS1, Tat(48-60), pVEC, MAP, and MTS.

The disclosure therefore relates to methods of treating ischemic injury in the central nervous system comprising administering, intranasally, an effective amount of an apoptotic target inhibitor to a subject in need thereof, wherein the ischemic injury is treated by such administration.

The disclosure relates to methods of treating ischemic injury in the central nervous system comprising administering, intranasally, an effective amount of an apoptotic target inhibitor to a subject in need thereof, wherein the apoptotic target inhibitor is conjugated to a cell-penetrating peptide.

The disclosure relates to methods of treating ischemic injury in the central nervous system comprising administering, intranasally, an effective amount of an apoptotic target inhibitor to a subject in need thereof, wherein the cell-penetrating peptide is selected from the group consisting of penetratin1, transportan, pIS1, Tat(48-60), pVEC, MAP, and MTS.

The apoptotic target inhibitor may be a caspase inhibitor, such as, but not limited to, an inhibitor of a caspase selected from the group consisting of caspase - 1, -2, -3, -4, -5, -6, -7, -8, -9, -10, -11, -12, and -14.

The disclosure relates to methods of treating ischemic injury in the central nervous system comprising administering intranasally, to a subject in need of such treatment, an effective amount of an apoptotic target inhibitor, wherein the apoptotic target inhibitor is a caspase-9 inhibitor and the administration occurs between the onset of the ischemic injury and 24hours post reperfusion. Clearance of the occlusion, which leads to onset of reperfusion, is common in clinical ischemia through medical intervention (tPA) or natural disruption. Reperfusion injury is a direct, frequent result of occlusion removal contributing disease burden by triggering apoptosis in the brain. In light of this, the transient occlusion model described herein takes into account damage caused by reperfusion and therefore the instant studies are labeled with their reperfusion timepoints. However, timing could equally be determined by other means, for example, but not limited to, measuring from onset of the ischemic injury.

The disclosure relates to methods of treating ischemic injury in the central nervous system comprising administering, intranasally, to a subject in need of such treatment, an effective amount of an apoptotic target inhibitor, wherein the apoptotic target inhibitor is a caspase-6 inhibitor and the administration occurs between 12 and 24 hours post reperfusion.

The disclosure relates to methods of inhibiting apoptosis in the central nervous system comprising administering, intranasally, an effective amount of an apoptotic target inhibitor to a subject in need thereof. For example, such inhibition is a modality of treating a neurodegenerative condition associated with apoptosis in the central nervous system, such as Alzheimer's Disease, Mild Cognitive Impairment, Parkinson's Disease, amyotrophic lateral sclerosis, Huntington's chorea, Creutzfeld-Jacob disease, etc. The apoptotic target inhibitor may be conjugated to a cell-penetrating peptide such as, but not limited to, penetratin1, transportan, pIS1, Tat(48-60), pVEC, MAP, or MTS, and/or the apoptotic target inhibitor is a caspase inhibitor, such as, but not limited to, an inhibitor of a caspase selected from the group consisting of caspase -1, -2, -3, -4, -5, -6, -7, -8, -9, -10, -11, -12, and -14 (preferably, but not by limitiation, an inhibitor of caspase 6 or 9).

The disclosure relates to compositions comprising an apoptotic target inhibitor conjugated to a cell-penetrating peptide.

The disclosure relates to compositions comprising an apoptotic target inhibitor conjugated to a cell-penetrating peptide, wherein the cell-penetrating peptide is selected from the group consisting of penetratin1, transportan, pIS1, Tat(48-60), pVEC, MAP, and MTS.

The disclosure relates to compositions comprising an apoptotic target inhibitor conjugated to a cell-penetrating peptide, wherein the apoptotic target inhibitor is a caspase inhibitor.

The disclosure relates to compositions comprising an apoptotic target inhibitor conjugated to a cell-penetrating peptide, wherein the apoptotic target inhibitor is selected from the group consisting of caspase -1, -2, -3, -4, -5, -6, -7, -8, - 9, -10, -11, -12, and -14 inhibitors. The disclosure relates to compositions comprising an apoptotic target inhibitor conjugated to a cell-penetrating peptide, wherein the apoptotic target inhibitor is a caspase inhibitor that specifically inhibits one caspase selected from the group consisting of caspase -1, -2, -3, -4, -5, -6, -7, -8, -9, -10, -11, - 12, and -14.

The disclosure relates to compositions comprising an apoptotic target inhibitor conjugated to a cell-penetrating peptide, wherein the apoptotic target inhibitor is selected from the group consisting of a small molecule inhibitor, a polypeptide inhibitor, and a nucleic acid inhibitor.

### 4. BRIEF DESCRIPTION OF THE FIGURES

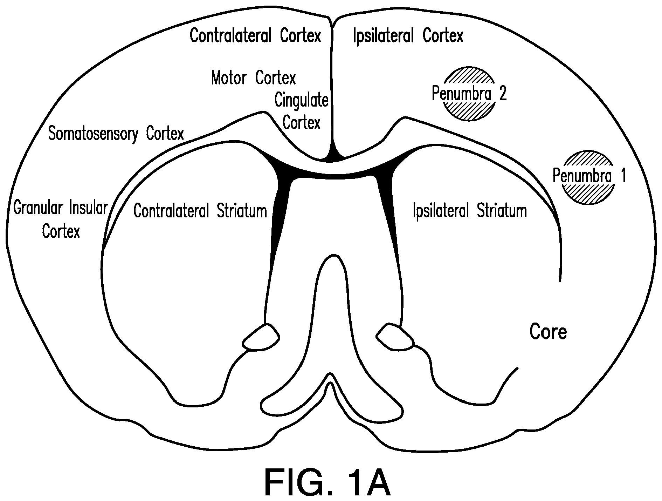

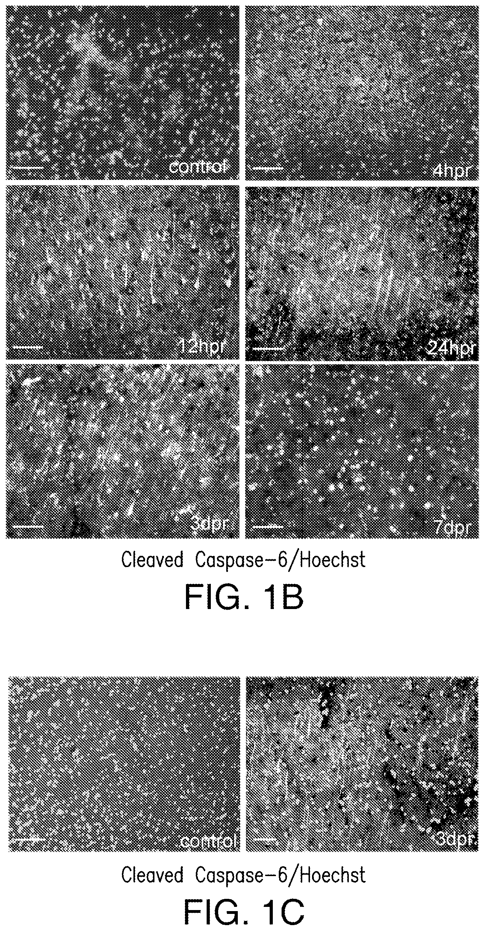

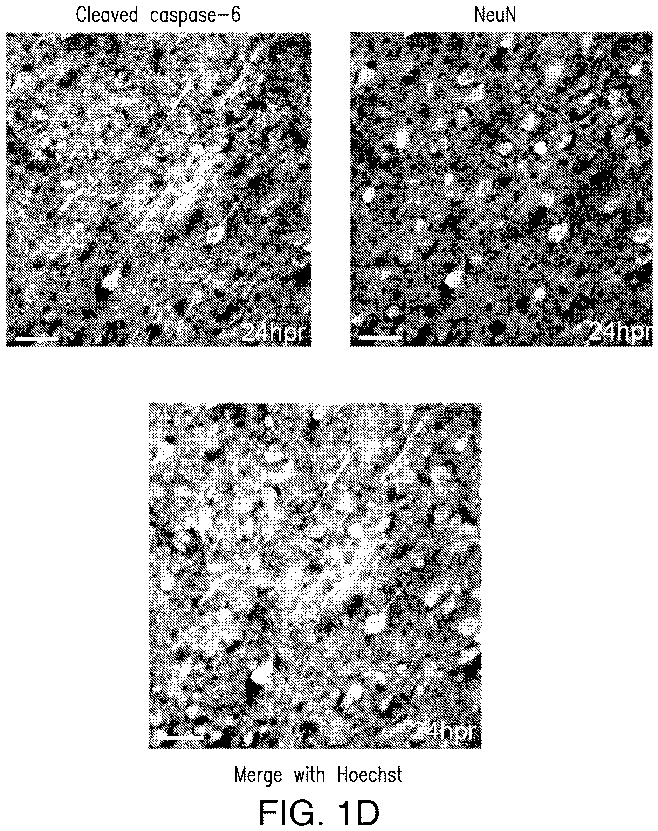

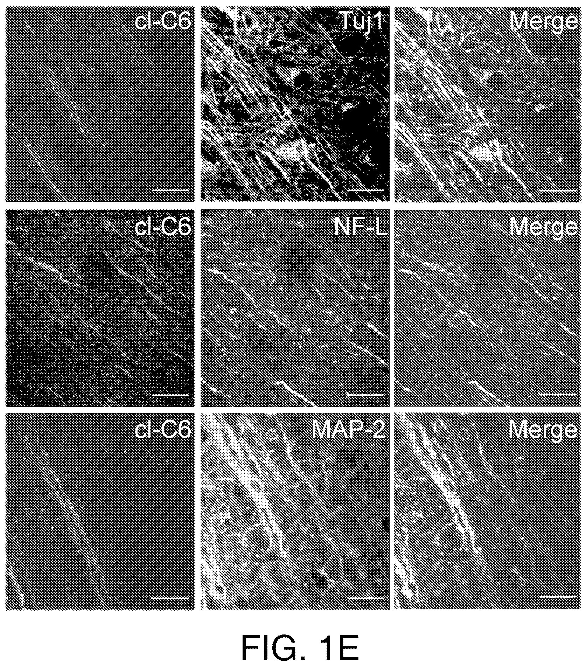

**Figure 1****. tMCAo induces activation of caspase-6 in neuronal processes and soma. 1a.** Schematic of core and penumbra region based on neuron density in fronto-corticostriatal region. Staining regions of interest in this figure and the remaining figures are of cortical layers III-IV of the cingulate, primary motor, primary + secondary somatosensory, and granular insular cortices in the penumbra. Ipsilateral hemisphere has had its MCA transiently occluded whereas the contralateral side has not been manipulated. **1b**. Rat tMCAo induces cleaved caspase-6 in cell bodies and processes in stroke penumbra. Rats were subjected to 2hr transient Middle Cerebral Artery Occlusion (tMCAo) followed by reperfusion for the indicated duration. Animals were perfused/fixed, brains sectioned and immunostained for cleaved caspase-6 (cl-C6, green) and nuclei were stained with Hoechst (blue). C1-C6 appears in cell bodies and processes at 12hr post-reperfusion (12hpr). Cell body and process staining is observed through 3 days post-reperfusion (3dpr). By 7dpr, nuclei and cell structures that resemble apoptotic bodies are positive for cl-C6. Scale bar: 50 µm. **1c.** Mouse tMCAo induces cleaved caspase-6 in cell bodies and processes in stroke penumbra. Mice were subjected to 1hr tMCAo and 3dpr. Animals were perfused, brains sectioned and immunostained for cl-C6 (green) and nuclei were stained with Hoechst (blue). C1-C6 appears in processes. Epifluorescence microscopy; Scale bar: 50 µm. **1d**. Cleaved Caspase-6 is neuron specific. Cortical penumbra tissue (layers III-IV) sections from stroked rats subject to tMCAo (24hpr) were immunostained with cl-C6 (green), NeuN (red), a neuronal marker, and Hoechst (blue). Left panel shows cl-C6, middle panel shows NeuN and right panel shows the merge of cl-c6, NeuN and Hoechst. C1-C6 does not co-localize with the astrocyte marker GFAP. Confocal microscopy; Scale bar: 50 µm. **1e**. Cleaved Caspase-6 is present in axons and dendrites. Upper panels: Cortical penumbra sections from stroked rats (12hpr) were immunostained with cl-C6 (red) and Tuj 1 (green), an axonal marker, and imaged using confocal microscopy. Left panel shows cl-C6, middle panel shows Tuj 1 and right panel shows a merge of both. Single processes that contain cl-C6 are apparent. Regions of axons with non-fragmented Tuj 1 staining do not have cl-C6 staining. In contrast, regions with cl-C6 exhibited fragmented Tuj1 staining. Middle panels: Brain sections were immunostained for cl-C6 (red) and the Neurofilament-Light chain (NF-L, green), another axon marker. Left panel shows cl-C6, middle panel shows NF-L and right panel shows a merge of both. The staining pattern is similar to cl-C6 and Tuj1: regions of axons with non-fragmented NF-L staining do not have cl-C6 staining. In contrast, regions with cl-C6 exhibited fragmented NF-L staining. Lower panels: Brains sections were immunostained with cl-C6 (red) and MAP-2 (green), a dendritic marker, and imaged using confocal microscopy. Left panel show cl-C6, middle panel show MAP-2 and right panel shows a merge of both. The pattern is similar to that observed with the axonal markers. Regions of axons with non-fragmented MAP-2 staining do not exhibit cl-C6 staining. In contrast, regions with cl-C6 exhibited fragmented MAP-2 staining. Confocal microscopy; Scale bar: 25 µm.

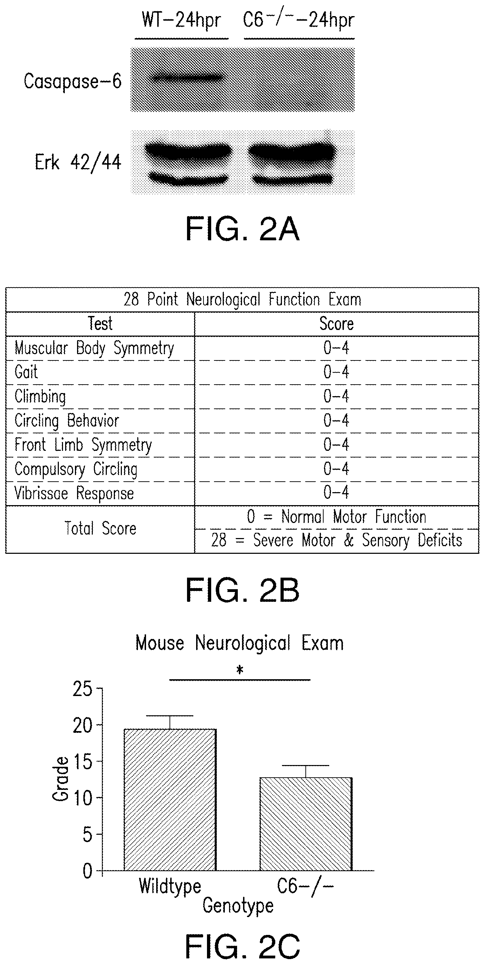

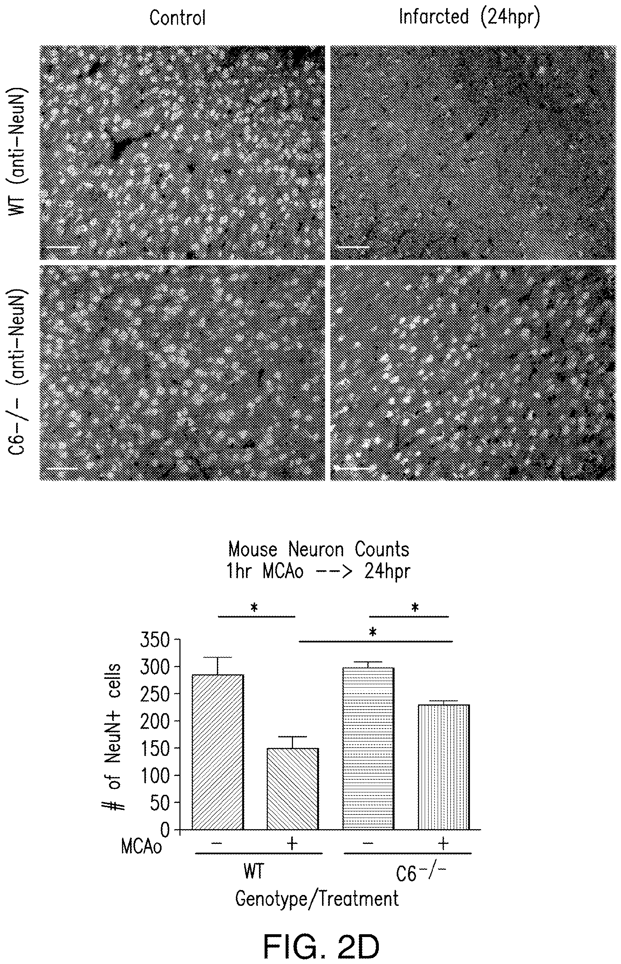

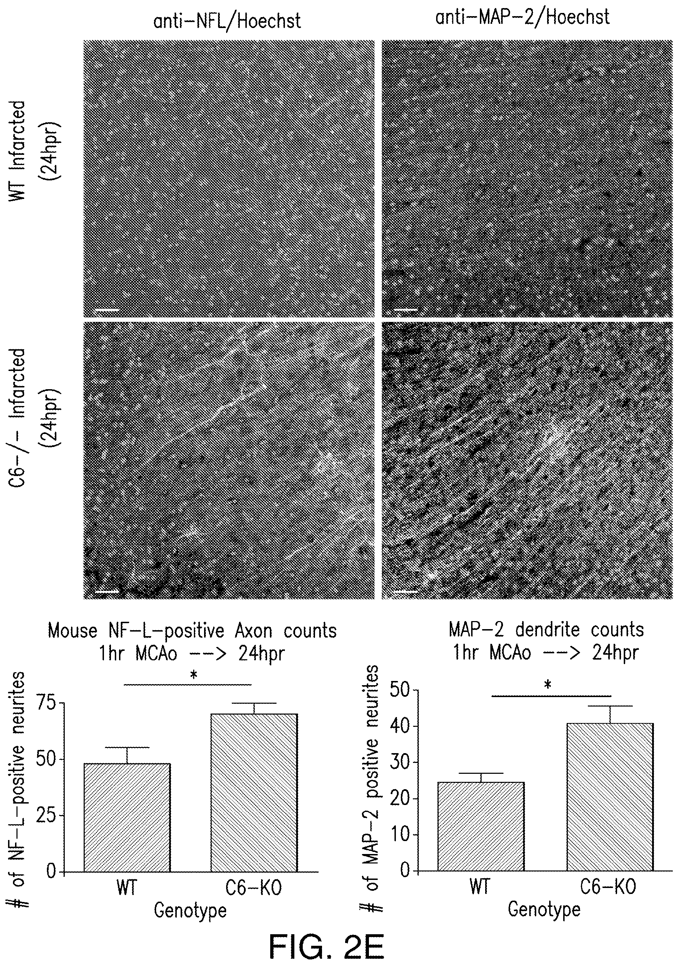

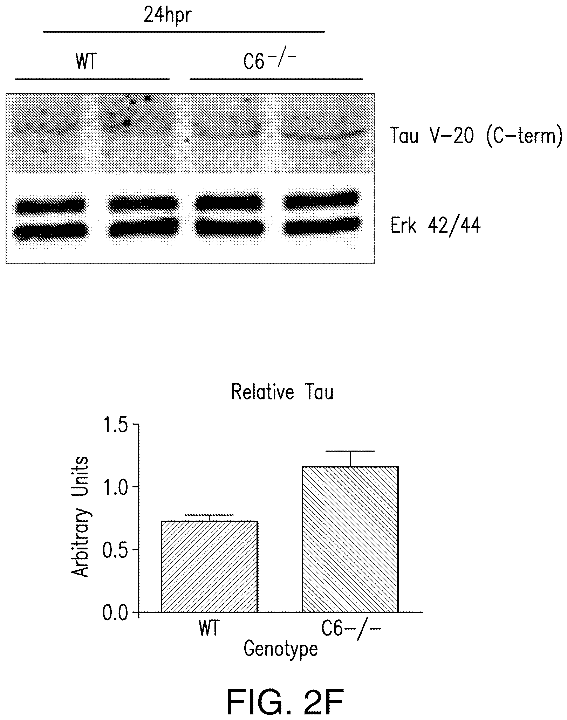

**Figure 2****. Caspase-6 knockout mice demonstrate retention of processes and neurons and improved neurological function following tMCAo. 2a.** Characterization of caspase-6^{-/-} mice. Western blot analysis of caspase-6 expression in wild-type and caspase-6^{-/-} mouse spleen. Erk expression is utilized as a loading control. **2b.** Criteria for Mouse Neurofunctional Exam. **2c.** Caspase-6 knockout improves neurologic function. Neurofunctional analysis score of wild-type and caspase-6^{-/-} mice following 1hr tMCAo and 24hpr. Caspase-6^{-/-} mice significantly outperform wild-type mice at 24hpr on the motor/ coordination tasks outlined in Table 1. Wild-type: 19.21 ± 1.931, n=14; caspase-6^{-/-}: 12.64 ± 1.525, n=14, p-value=0.0129. **2d.** Caspase-6 knockout preserves neurons. Wild-type and caspase-6^{-/-} mice were subjected to 1hr tMCAo and sacrificed at 24hpr. NeuN staining of brain sections reveals a significant decrease in the number of neurons in stroked wild-type mice (148.0 ± 20.22, n=3) compared to non-infarcted wild-type mice (282.7 ± 32.97, n=3; p=0.0253). Caspase-6^{-/-} mice subjected to tMCAo retain more neurons than stroked wild-type mice (225.0 ± 8.114, n=4 vs. 148.0 ± 20.22, n=3; p=0.0108). Non-stroked wild-type and nonstroked caspase-6^{-/-} mice have a statistically insignificant difference in the number of neurons (282.7 ± 32.97, n=3 vs. 296.3 ± 9.207, n=3). Epifluorescence microscopy; Scale bar: 50 µm. Cortical penumbra tissue staining. Nissl staining yielded similar results. **2e.** Caspase-6 knockout preserves neuronal processes. Brain sections from wildtype and caspase-6^{-/-} mice subjected to 1hr tMCAo and 24hpr were immunostained for NF-L (upper panels) and MAP-2 (lower panels). Stroked wild-type mice have fewer NFL and MAP-2 positive processes compared to stroked caspase-6^{-/-} mice (47.67 ± 7.219, n=3 vs. 70.00 ± 4.916, n=4; p=0.0447). NF-L positive processes were also shorter and more fragmented in wild-type mice compared to caspase-6^{-/-}. Reduction in MAP-2 positive neurites (dendrites) is observed with stroked wild-type mice. Wild-type: 24.00 ± 2.887, n=3, caspase-6^{-/-}: 40.33 ± 4.807, n=3. Epifluorescence microscopy; Scale bar: 50µm. Cortical penumbra tissue staining. **2f.** Caspase-6 knockout prevents reduction in tau. Brain lysates from wild-type and caspase-6^{-/-} mice subjected to 1hr tMCAo and 24hpr were isolated and analyzed by western blot. Tau expression was analyzed with anti-Tau (V-20), which recognizes the C-terminal end of Tau, the putative location of a caspase-6 cleavage site. (Guo, et al., Am J Pathol 165 (2), 523-531 (2004); Horowitz, et al., J Neurosci 24 (36), 7895-7902 (2004)). Stroked caspase-6^{-/-} mice contain more tau than stroked wild-type mice. Erk was used as a loading control and normalization (n=2). Densitometry was performed with gel analysis from Image J. Error bars are standard deviation.

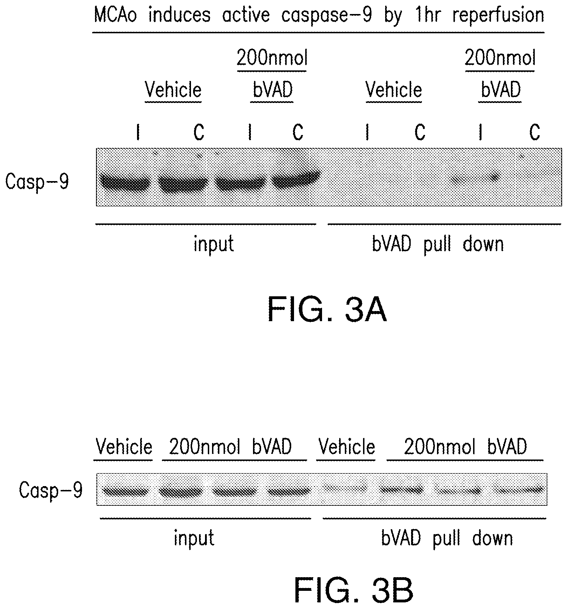

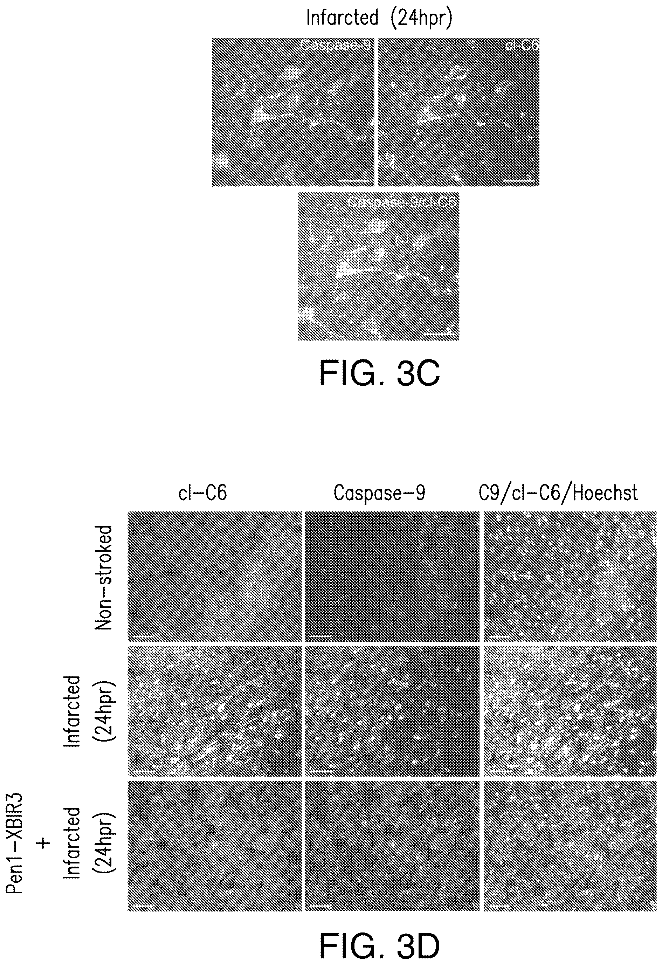

**Figure 3****. Caspase-9 is active early in stroke and co-localizes with cl-C6. 3a.** Active caspase-9 is induced in the stroke core by tMCAo within 1hpr. bVAD-fmk was infused with ICC into the predicted stroke area of rats prior to tMCAo. Animals were harvested at 1hpr and bVAD-caspase complexes isolated and analyzed by western blotting. I-ipsilateral, C-contralateral. 3b. Active caspase-9 continues to be activated in stroke. VAD-fmk was infused with ICC and animals were harvested at 4hpr and bVAD-caspase complexes were isolated and analyzed by Western blotting.. **3c.** Caspase-9 and cleaved caspase-6 are induced in the same cells following tMCAo. Rats were subjected to 2hr tMCAo followed by 24hpr. Confocal analysis of caspase-9 and cl-C6 immunostaining reveals cells co-labeled with caspase-9 and cl-C6 at 24hpr. Caspase-9 is visible in the processes along with cl-C6. Normal tissue from rodents not subjected to tMCAo does not display caspase-9 or cl-C6 staining (Figure 3C). Confocal microscopy; Scale bar: 25 µm. Cortical penumbra tissue staining. **3d.** Pen1-XBIR3 blocks tMCAo induction of cleaved caspase-6 in neuronal soma and processes. Rats were treated with Pen1-XBIR3 or vehicle prior to tMCAo and harvested at 24hpr for immunohistochemistry for cl-C6 (green), caspase-9 (red) and Hoechst (blue). Upper panels show a non-stroked animal. Middle panels show vehicle and lower panels show a Pen1-XBIR3 treated animal. The caspase-9 specific inhibitor, Pen1-XBIR3, blocks the increase in caspase-9 and the induction of cl-C6 observed at 24hpr. Epifluorescence microscopy. Scale bar: 50 µm.

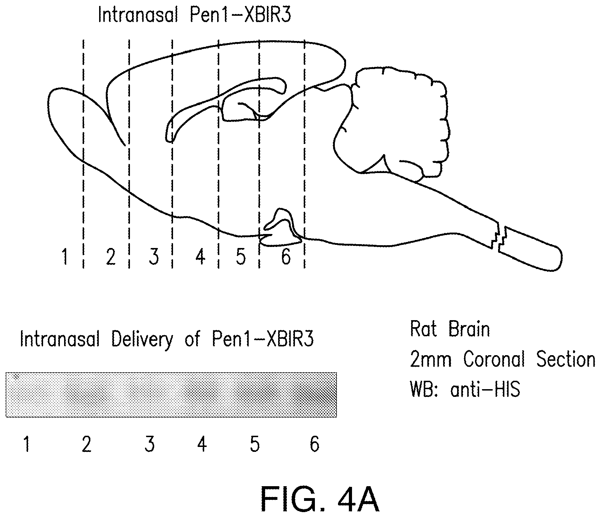

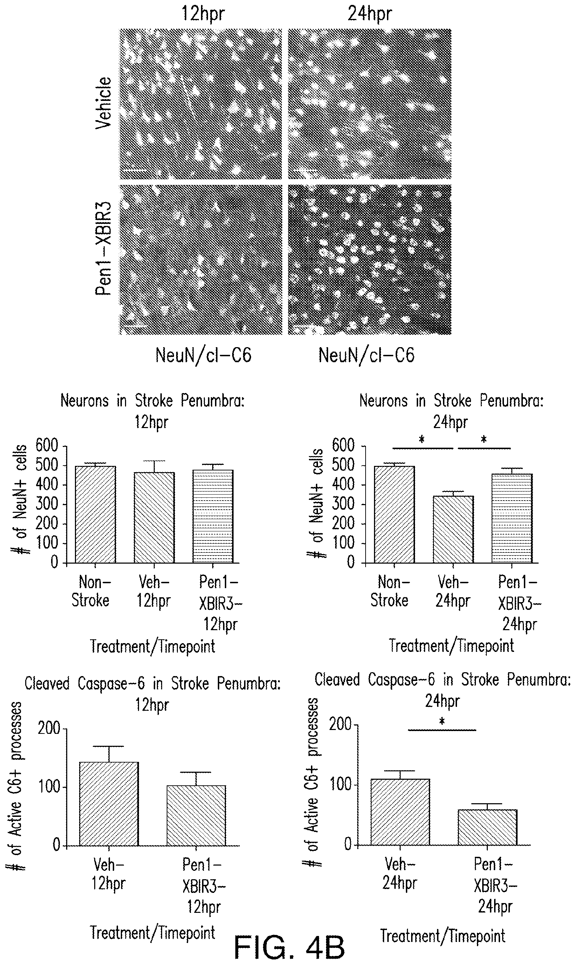

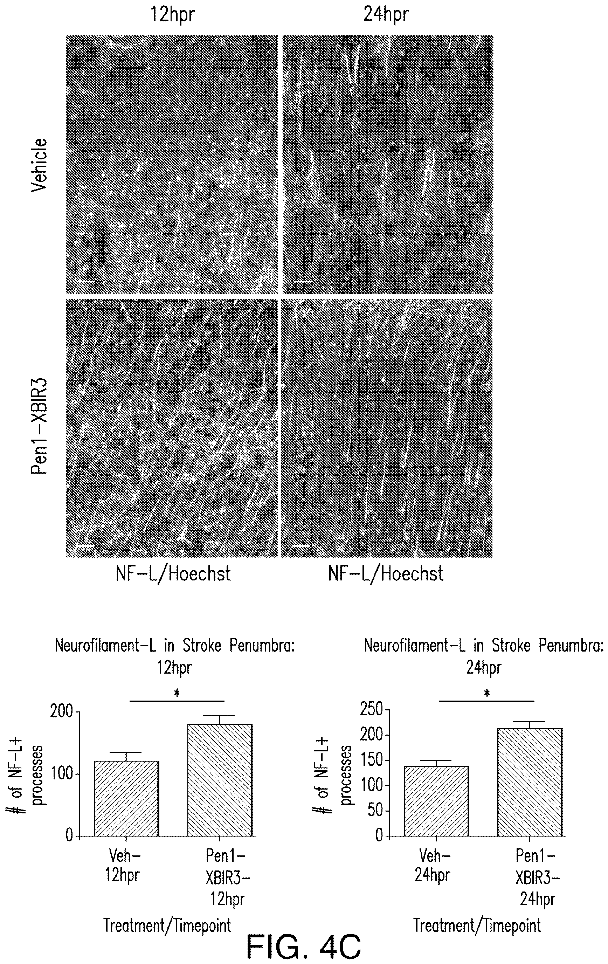

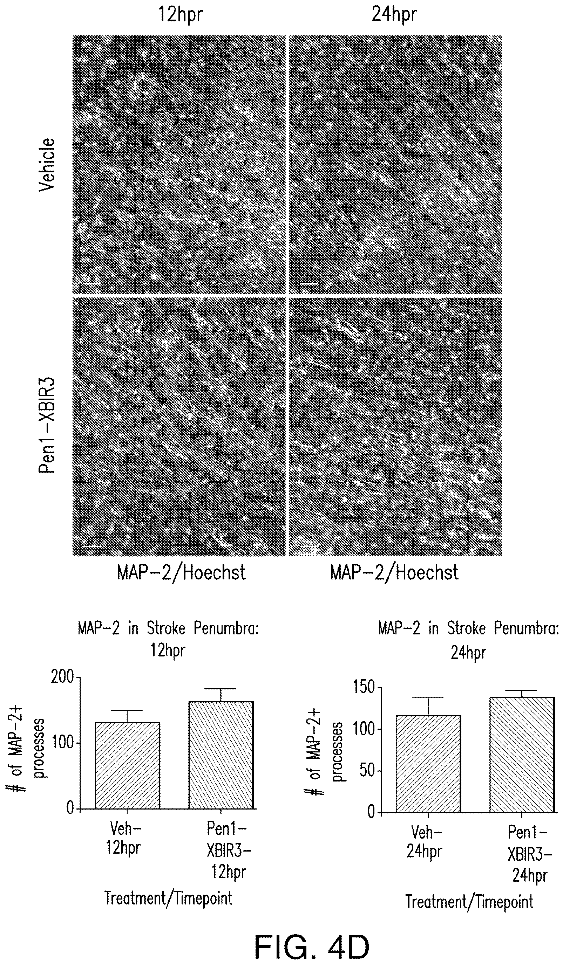

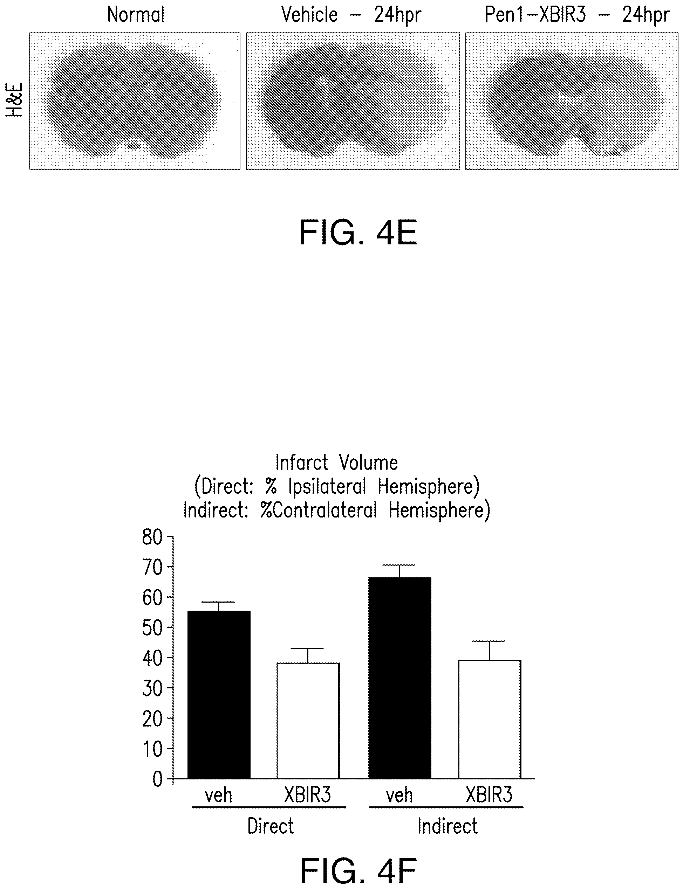

**Figure 4****. Intranasal delivery of Pen1-XBIR3 ameliorates caspase-6 activation in neurites and abrogates loss of processes. 4a.** Intranasal application delivers Pen1-XBIR3 throughout the rat CNS. The injected rat was sacrificed 1hr after intranasal delivery of Pen1-XBIR3 (60µl). The brain was sliced into 6 - 2mm coronal sections from anterior (olfactory bulbs) to posterior (occipital pole). Slices were solubilized and protein analyzed by SDS-PAGE and western blotting with anti-HIS, to visualize XBIR3. Lanes 1-6 coronal sections anterior (1) to posterior (6), as indicated on schematic. **4b.** Intranasal Pen1-XBIR3 protects neurons and decreases cleaved caspase-6 in processes at 24hpr. Vehicle or Pen1-XBIR3 was delivered intranasally prior to tMCAo and rats were harvested at 12hpr (left panels) and 24hpr (right panels). Sections were immunostained for NeuN (green) and cl-C6 (red) and NeuN positive cells and cl-C6 positive processes were quantified. NeuN: Non-stroked: 494.7±18.52, n=3; Vehicle-12hpr: 463.7±57.53, n=3; Pen1-XBIR3-12hpr: 477.3±28.95, n=3; Vehicle-24hpr: 338.0±22.91, n=3; Pen1-XBIR3-24hpr: 453.7±25.44, n=3; Vehicle vs. Pen1-XBIR3-24hpr p-value: 0.0278. C1-C6 processes: Vehicle-12hpr: 144.0±28.50, n=3 vs. Pen1-XBIR3-12hpr: 102.7±23.15, n=3; p=0.3233. Vehicle-24hpr: 109.7±12.73, n=3 vs. Pen1-XBIR3-24hpr: 57.33±10.04, n=3; p=0.032. Epifluorescence microscopy. Scale bar: 50 µm. Nissl staining yielded similar results to NeuN. **4c.** Intranasal Pen1-XBIR3 blocks the reduction in NF-L positive processes induced by tMCAo in rats. Rats were treated as in B and sections were immunostained for NF-L (green) and NF-L positive axons were quantified at 12 and 24hpr. Vehicle-12hpr: 118.7±14.88, n=3; Pen1-XBIR3-12hpr: 179.7±14.89, n=3; Vehicle-24hpr: 138.0±9.074, n=3; Pen1-XBIR3-24hpr: 213.7±11.84, n=3. Epifluorescence microscopy. Scale bar: 50 µm. **4d.** Intranasal Pen1-XBIR3 does not affect the number of MAP-2 positive processes (dendrites) associated with tMCAo in rats. Rats were treated as in B and sections were immunostained for MAP-2 (green) and MAP-2 positive axons were quantified at 12 and 24hpr. Vehicle-12hpr: 130.3±18.26, n=3; Pen1-XBIR3-12hpr: 162.0±19.22, n=3; Vehicle-24hpr: 116.7±19.33, n=3; Pen1-XBIR3-24hpr: 138.3±6.766, n=3. Epifluorescence microscopy. Scale bar: 50 µm. **4e.** Intranasal Pen1-XBIR3 reduces ischemic infarct volume. Vehicle or Pen1-XBIR3 was delivered intranasally and rats were harvested at 24 hpr. Sections were stained with H&E. **4f.** Direct (infarct area/ipsilateral hemisphere area) and indirect (infarct area/contralateral hemisphere) stroke volumes were quantified, n = 3 (ANOVA, p<0.05).

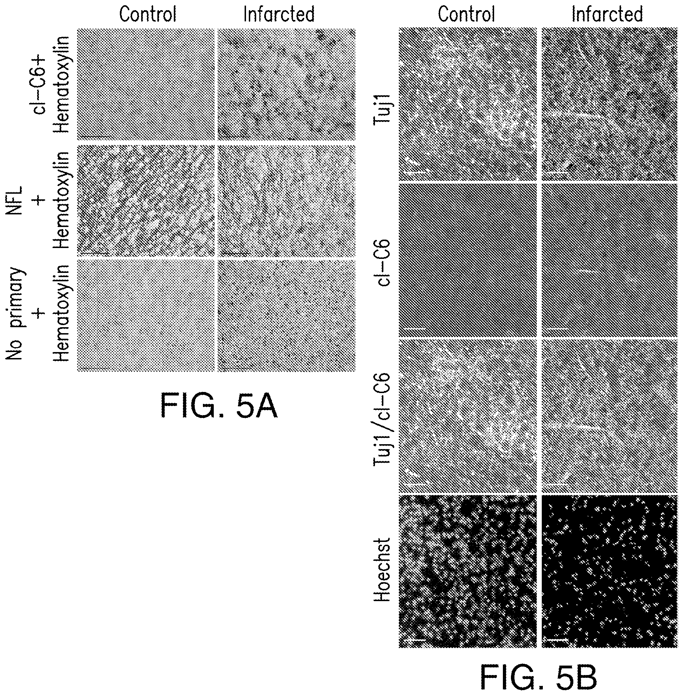

**Figure 5****. Active caspase-6 in human ischemia. Post-mortem brain tissue from a patient who had suffered an infarct, as compared to brain tissue from an age-matched control. 5a.** Immuno-histological analysis (DAB processing) for cleaved caspase-6. DAB processing for cl-C6 showed cell body and process staining. Sections stained without primary antibody show no cell body or process staining. Cleaved caspase-6 process staining resembles neurofilament-L process staining. Sections from age-matched control brain show no cleaved caspase-6 staining. Scale bar: 100 µm. **5b.** Immunofluorescent staining for cleaved caspase-6 and Tuj1. The infarct area shows the presence of cleaved caspase-6 in a process, Tuj1 appears in the same process. The control brain has no evidence of cleaved caspase-6. Epifluorescence microscopy; Scale bar: 50 µm

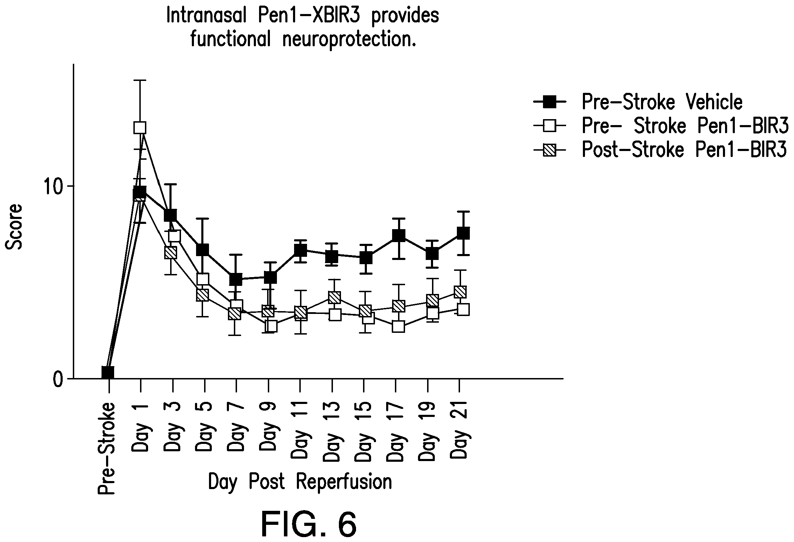

**Figure 6****. Intranasal Penl-XBIR3 provides long-term protection from stroke.** 2hr tMCAo was performed on rats given either prophylactic (pre-stroke) intranasal vehicle (black squares) or prophylactic (pre-stroke) (blue triangles)/therapeutic (post-stroke) (red circles) Pen1-XBIR3. Rats were monitored for 21 days. Means (with SEM) of neurofunctional score. *p < 0.05.

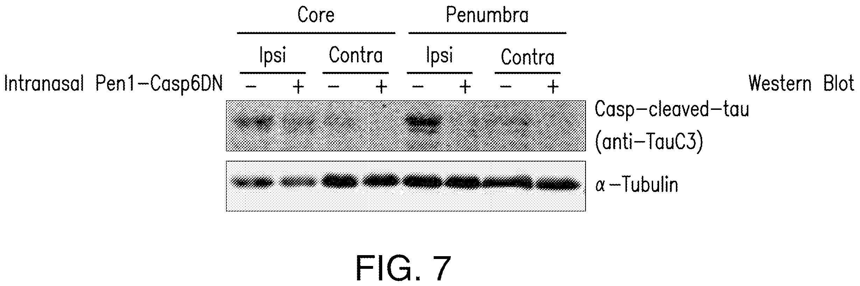

**Figure 7****. IntranasalPenl-C6DN prevented the cleavage of caspase-6 substrates during stroke.** Protein lysate from the core and penumbra regions of the stroke infarct (24hpr) was isolated. Ipsilateral (stroked) hemispheres contained abundant caspase-cleaved tau when only treated with vehicle. Pen1-C6DN reduced cleavage of caspase-cleaved tau.

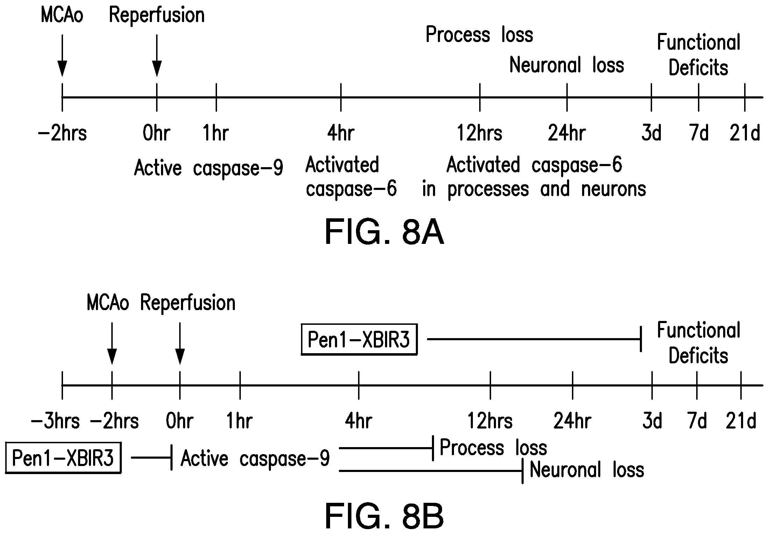

**Figure 8****. Schematic representation of tMCAo mechanistic and functional timeline.** 8a. Molecular and functional effects of tMCAo. 8b. Intervention with Pen1-XBIR3 prophylactically at 3 h inhibits active caspase-9, blocks activation of caspase-6, and prevents process and neuronal loss. Intervention with Pen1-XBIR3 therapeutically at 4 hpr provides functional recovery up to 21 d.

### 5. DETAILED DESCRIPTION OF THE INVENTION

The present invention relates to compositions for use in the treatment of ischemic injury in the central nervous system ("CNS"). Thus, disclosed herein are compositions and methods useful for extending the therapeutic window associated with CNS ischemic injury by inhibiting particular apoptotic targets that are either expressed or activated at certain time points after the occurrence of the CNS ischemic injury.

### 5.1 Apoptotic Target Inhibtor Compositions

### 5.1.1 Caspase Inhibitors

The disclosure relates to inhibitors of apoptosis, such as, but not limited to, compositions that inhibit the apoptotic activity of certain apoptosis-inducing targets. Such apoptotic targets include, but are not limited to, members of the caspase family of proteins. Caspases appear to follow a hierarchical order of activation starting with extrinsic (originating from extracellular signals) or intrinsic apoptotic signals which trigger the initiator group (caspase- 8, 10, 9 or 2) which in turn process the executioner caspases (caspase- 7, 3 and 6). Initiator or executioner or both classes of caspases may be inhibited according to the invention. For example, but not by way of limitation, the inhibitors of the instant invention target one or more of caspases -1, -2, -3, -4, -5, -6, -7, -8, -9, 10, -11, -12, and -14. The inhibitor may be a non-specific inhibitor of one or more of caspases -1, -2, -3, -4, -5, -6, -7, -8, -9, 10, - 11, -12, and -14. The inhibitor may be a specific inhibitor of a single caspase or of a particular subset of caspases selected from the group consisting of caspases -1, -2, -3, -4, -5, -6, -7, -8, -9, 10, -11, -12, and -14. The specific inhibitor may be an inhibitor of caspase-9 or inhibitor of caspase-6.

The apoptotic target inhibitors may include, but are not limited to, caspase inhibitors, are selected from the group consisting of small molecule inhibitors, peptide/protein inhibitors, and nucleic acid inhibitors. Such inhibitors can exert their function by inhibiting either the expression or activity of an apoptotic target.

The apoptotic target inhibitors may include small molecule inhibitors of caspases. The small molecule inhibitors of caspases may include, but are not limited to, isatin sulfonamides (Lee, et al., J Biol Chem 275:16007-16014 (2000); Nuttall, et al., Drug Discov Today 6:85-91 (2001)), anilinoquinazolines (Scott, et al., JPET 304 (1) 433-440 (2003), and one or more small molecule caspase inhibitor disclosed in U.S. Patent No. 6,878,743.

The apoptotic target inhibitors may be peptide inhibitors of caspases. The peptide inhibitors of caspases may include, but are not limited to EG Z-VEID-FMK (WO 2006056487); Z-VAD-FMK, CrmA, and Z-VAD-(2, 6-dichlorobenzoyloxopentanoic acid) (Garcia-Calvo, et al., J. Biol. Chem., 273, 32608-32613 (1998)).

The apoptotic target inhibitors may include, but are not limited to the class of protein inhibitors identified as Inhibitors of Apoptosis ("IAPs"). IAPs generally contain one to three BIR (baculovirus IAP repeats) domains, each consisting of approximately 70 amino acid residues. In addition, certain IAPs also have a RING finger domain, defined by seven cysteines and one histidine (e.g. C3HC4) that can coordinate two zinc atoms. Exemplary mammalian IAPs, such as, but not limited to c-IAP1 (Accession No. Q13490.2), cIAP2 (Accession No. Q13489.2), and XIAP (Accession No. P98170.2), each of which have three BIRs in the N-terminal portion of the molecule and a RING finger at the C-terminus. In contrast, NAIP (Accession No. Q13075.3), another exemplary mammalian IAP, contains three BIRs without RING, and survivin (Accession No. 015392.2) and BRUCE (Accession No. Q9H8B7), which are two additional exemplary IAPs, each has just one BIR.

The apoptotic target inhibitor may be a dominant negative form of a caspase polypeptide. For example, but not by way of limitation, the dominant negative form of a caspase polypeptide can be a dominant negative form of caspase-6. The dominant negative form of caspase-6 may be the polypeptide designated "C6DN" in Denault, J.B. and G.S. Salvesen, Expression, purification, and characterization of caspases. Curr Protoc Protein Sci, 2003. Chapter 21: p. Unit 21 13. The dominant negative form of a caspase polypeptide may be a dominant negative form of a caspase selected from the group consisting of caspases -1, -2, -3, -4, -5, -7, -8, -9, 10, -11, -12, and -14.

Polypeptide apoptotic target inhibitors include those amino acid sequences that retain certain structural and functional features of the identified apoptotic target inhibitor polypeptides, yet differ from the identified inhibitors' amino acid sequences at one or more positions. Such polypeptide variants can be prepared by substituting, deleting, or adding amino acid residues from the original sequences via methods known in the art.

Such substantially similar sequences may include sequences that incorporate conservative amino acid substitutions. As used herein, a "conservative amino acid substitution" is intended to include a substitution in which the amino acid residue is replaced with an amino acid residue having a similar side chain. Families of amino acid residues having similar side chains have been defined in the art, including: basic side chains (e.g., lysine, arginine, histidine); acidic side chains (e.g., aspartic acid, glutamic acid); uncharged polar side chains (e.g., glycine, asparagine, glutamine, serine, threonine, tyrosine, cysteine); nonpolar side chains (e.g., alanine, valine, leucine, isoleucine, proline, phenylalanine, methionine, tryptophan); β-branched side chains (e.g., threonine, valine, isoleucine); and aromatic side chains (e.g., tyrosine, phenylalanine, tryptophan, histidine). Other generally preferred substitutions involve replacement of an amino acid residue with another residue having a small side chain, such as alanine or glycine. Amino acid substituted peptides can be prepared by standard techniques, such as automated chemical synthesis.

The polypeptide disclosed hereinmay be at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% homologous to the amino acid sequence of the original apoptotic target inhibitor, such as an IAP, and is capable of apoptotic target inhibition. As used herein, the percent homology between two amino acid sequences may be determined using standard software such as BLAST or FASTA. The effect of the amino acid substitutions on the ability of the synthesized polypeptide to inhibit apoptotic targets can be tested using the methods disclosed in Examples section, below.

For example, but not by way of limitation, the apoptotic target inhibitors of the instant invention which are nucleic acids include, but are not limited to, inhibitors that function by inhibiting the expression of the target, such as ribozymes, antisense oligonucleotide inhibitors, and siRNA inhibitors. A "ribozyme" refers to a nucleic acid capable of cleaving a specific nucleic acid sequence. A ribozyme may be understood to refer to RNA molecules that contain anti-sense sequences for specific recognition, and an RNA-cleaving enzymatic activity, see, for example, U.S. Pat. No. 6,770,633. In contrast, "antisense oligonucleotides" generally are small oligonucleotides complementary to a part of a gene to impact expression of that gene. Gene expression can be inhibited through hybridization of an oligonucleotide to a specific gene or messenger RNA (mRNA) thereof. In some cases, a therapeutic strategy can be applied to dampen expression of one or several genes believed to initiate or to accelerate inflammation, see, for example, U.S. Pat. No. 6,822,087 and WO 2006/062716. A "small interfering RNA" or "short interfering RNA" or "siRNA" or "short hairpin RNA" or "shRNA" are forms of RNA interference (RNAi). An interfering RNA can be a double-stranded RNA or partially double-stranded RNA molecule that is complementary to a target nucleic acid sequence, for example, caspase 6 or caspase 9. Micro interfering RNA's (miRNA) also fall in this category. A double-stranded RNA molecule is formed by the complementary pairing between a first RNA portion and a second RNA portion within the molecule. The length of each portion generally is less than 30 nucleotides in length (e.g., 29, 28, 27, 26, 25, 24, 23, 22, 21, 20, 19, 18, 17, 16, 15, 14, 13, 12, 11, or 10 nucleotides). The length of each portion may be 19 to 25 nucleotides in length. In some siRNA molecules, the complementary first and second portions of the RNA molecule are the "stem" of a hairpin structure. The two portions can be joined by a linking sequence, which can form the "loop" in the hairpin structure. The linking sequence can vary in length. The linking sequence can be 5, 6, 7, 8, 9, 10, 11, 12 or 13 nucleotides in length. Linking sequences can be used to join the first and second portions, and are known in the art. The first and second portions are complementary but may not be completely symmetrical, as the hairpin structure may contain 3' or 5' overhang nucleotides (e.g., a 1, 2, 3, 4, or 5 nucleotide overhang). The RNA molecules of the invention can be expressed from a vector or produced chemically or synthetically.

### 5.1.2 Apoptosis Inhibitor-Cell Penetrating Peptide Conjugates

The apoptotic target inhibitor may be conjugated to a cell penetrating peptide to form an Apoptosis Inhibitor-Cell Penetrating Peptide {"AICPP") conjugate. The AICPP conjugate can facilitate delivery of the inhibitor to into a cell in which it is desirable to prevent apoptosis.

As used herein, a "cell-penetrating peptide" is a peptide that comprises a short (about 12-30 residues) amino acid sequence or functional motif that confers the energy-independent (i.e., non-endocytotic) translocation properties associated with transport of the membrane-permeable complex across the plasma and/or nuclear membranes of a cell. The cell-penetrating peptide used in the membrane-permeable complex preferably comprises at least one non-functional cysteine residue, which is either free or derivatized to form a disulfide link with the apoptotic target inhibitor, which has been modified for such linkage. Representative amino acid motifs conferring such properties are listed in U.S. Pat. No. 6,348,185. The cell-penetrating peptides of the present invention preferably include, but are not limited to, penetratin1, transportan, pIs1, TAT(48-60), pVEC, MTS, and MAP.

The cell-penetrating peptides of the present invention include those sequences that retain certain structural and functional features of the identified cell-penetrating peptides, yet differ from the identified peptides' amino acid sequences at one or more positions. Such polypeptide variants can be prepared by substituting, deleting, or adding amino acid residues from the original sequences via methods known in the art.

Such substantially similar sequences include sequences that incorporate conservative amino acid substitutions, as described above in connection with polypeptide apoptotic target inhibitors. A cell-penetrating peptide may be at least about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% homologous to the amino acid sequence of the identified peptide and is capable of mediating cell penetration. The effect of the amino acid substitutions on the ability of the synthesized peptide to mediate cell penetration can be tested using the methods disclosed in Examples section, below.

The cell-penetrating peptide of the membrane-permeable complex may be penetratin1, comprising the peptide sequence RQIKIWFQNRRMKWKK, or a conservative variant thereof. As used herein, a "conservative variant" is a peptide having one or more amino acid substitutions, wherein the substitutions do not adversely affect the shape--or, therefore, the biological activity (i.e., transport activity) or membrane toxicity--of the cell-penetrating peptide.

Penetratin1 is a 16-amino-acid polypeptide derived from the third alpha-helix of the homeodomain of Drosophila antennapedia. Its structure and function have been well studied and characterized: Derossi et al., Trends Cell Biol., 8(2):84-87, 1998; Dunican et al., Biopolymers, 60(1):45-60, 2001; Hallbrink et al., Biochim. Biophys. Acta, 1515(2):101-09, 2001; Bolton et al., Eur. J. Neurosci., 12(8):2847-55, 2000; Kilk et al., Bioconjug. Chem., 12(6):911-16, 2001; Bellet-Amalric et al., Biochim. Biophys. Acta, 1467(1):131-43, 2000; Fischer et al., J. Pept. Res., 55(2): 163-72, 2000; Thoren et al., FEBS Lett., 482(3):265-68, 2000.

It has been shown that penetratin1 efficiently carries avidin, a 63-kDa protein, into human Bowes melanoma cells (Kilk et al., Bioconjug. Chem., 12(6):911-16, 2001). Additionally, it has been shown that the transportation of penetratin1 and its cargo is non-endocytotic and energy-independent, and does not depend upon receptor molecules or transporter molecules. Furthermore, it is known that penetratin1 is able to cross a pure lipid bilayer (Thoren et al., FEBS Lett., 482(3):265-68, 2000). This feature enables penetratin1 to transport its cargo, free from the limitation of cell-surface-receptor/-transporter availability. The delivery vector previously has been shown to enter all cell types (Derossi et al., Trends Cell Biol., 8(2):84-87, 1998), and effectively to deliver peptides (Troy et al., Proc. Natl. Acad. Sci. USA, 93:5635-40, 1996) or antisense oligonucleotides (Troy et al., J. Neurosci., 16:253-61, 1996; Troy et al., J. Neurosci., 17:1911-18, 1997).

The disclosure also relates to the use of the following exemplary cell permeant molecules: RL16 (H-RRLRRLLRRLLRRLRR-OH), a sequence derived from Penetratin1 with sightly different physical properties (Biochim Biophys Acta. 2008 Jul-Aug;1780(7-8):948-59); and RVGRRRRRRRRR, a rabies virus sequence which targets neurons see P. Kumar, H. Wu, J.L. McBride, K.E. Jung, M.H. Kim, B.L. Davidson, S.K. Lee, P. Shankar and N. Manjunath, Transvascular delivery of small interfering RNA to the central nervous system, Nature 448 (2007), pp. 39-43.

The cell-penetrating peptide of the membrane-permeable complex is a cell-penetrating peptides selected from the group consisting of: transportan, pIS1, Tat(48-60), pVEC, MAP, and MTS. Transportan is a 27-amino-acid long peptide containing 12 functional amino acids from the amino terminus of the neuropeptide galanin, and the 14-residue sequence of mastoparan in the carboxyl terminus, connected by a lysine (Pooga et al., FASEB J., 12(1):67-77, 1998). It comprises the amino acid sequence GWTLNSAGYLLGKINLKALAALAKKIL, or a conservative variant thereof.

pIs1 is derived from the third helix of the homeodomain of the rat insulin 1 gene enhancer protein (Magzoub et al., Biochim. Biophys. Acta, 1512(1):77-89, 2001; Kilk et al., Bioconjug. Chem., 12(6):911-16, 2001). pIs1 comprises the amino acid sequence PVIRVW FQNKRCKDKK, or a conservative variant thereof.

Tat is a transcription activating factor, of 86-102 amino acids, that allows translocation across the plasma membrane of an HIV-infected cell, to transactivate the viral genome (Hallbrink et al., Biochem. Biophys. Acta., 1515(2):101-09, 2001; Suzuki et al., J. Biol. Chem., 277(4):2437-43, 2002; Futaki et al., J. Biol. Chem., 276(8):5836-40, 2001). A small Tat fragment, extending from residues 48-60, has been determined to be responsible for nuclear import (Vives et al., J. Biol. Chem., 272(25):16010-017, 1997); it comprises the amino acid sequence GRKKRRQRRRPPQ, or a conservative variant thereof.

pVEC is an 18-amino-acid-long peptide derived from the murine sequence of the cell-adhesion molecule, vascular endothelial cadherin, extending from amino acid 615-632 (Elmquist et al., Exp. Cell Res., 269(2):237-44, 2001). pVEC comprises the amino acid sequence LLIILRRRIRKQAHAH, or a conservative variant thereof.

MTSs, or membrane translocating sequences, are those portions of certain peptides which are recognized by the acceptor proteins that are responsible for directing nascent translation products into the appropriate cellular organelles for further processing (Lindgren et al., Trends in Pharmacological Sciences, 21(3):99-103, 2000; Brodsky, J. L., Int. Rev. Cyt., 178:277-328, 1998; Zhao et al., J. Immunol. Methods, 254(1-2):137-45, 2001). An MTS of particular relevance is MPS peptide, a chimera of the hydrophobic terminal domain of the viral gp41 protein and the nuclear localization signal from simian virus 40 large antigen; it represents one combination of a nuclear localization signal and a membrane translocation sequence that is internalized independent of temperature, and functions as a carrier for oligonucleotides (Lindgren et al., Trends in Pharmacological Sciences, 21(3):99-103, 2000; Morris et al., Nucleic Acids Res., 25:2730-36, 1997). MPS comprises the amino acid sequence GALFLGWLGAAGSTMGAWSQPKKKRKV, or a conservative variant thereof.

Model amphipathic peptides, or MAPs, form a group of peptides that have, as their essential features, helical amphipathicity and a length of at least four complete helical turns (Scheller et al., J. Peptide Science, 5(4):185-94, 1999; Hallbrink et al., Biochim. Biophys. Acta., 1515(2):101-09, 2001). An exemplary MAP comprises the amino acid sequence KLALKLALKALKAALKLA-amide, or a conservative variant thereof.

The cell-penetrating peptides and the apoptotic target inhibitors described above are covalently bound to form AICPP conjugates. The cell-penetrating peptide is operably linked to a peptide apoptotic target inhibitor via recombinant DNA technology. Where the apoptotic target inhibitor is a peptide or polypeptide sequence, a nucleic acid sequence encoding that apoptotic target inhibitor can be introduced either upstream (for linkage to the amino terminus of the cell-penetrating peptide) or downstream (for linkage to the carboxy terminus of the cell-penetrating peptide), or both, of a nucleic acid sequence encoding the apoptotic target inhibitor of interest. Such fusion sequences comprising both the apoptotic target inhibitor encoding nucleic acid sequence and the cell-penetrating peptide encoding nucleic acid sequence can be expressed using techniques well known in the art.

The apoptotic target inhibitor can be operably linked to the cell-penetrating peptide via a non-covalent linkage. Such non-covalent linkage may be mediated by ionic interactions, hydrophobic interactions, hydrogen bonds, or van der Waals forces.

The apoptotic target inhibitor may be operably linked to the cell penetrating peptide via a chemical linker. Examples of such linkages typically incorporate 1-30 nonhydrogen atoms selected from the group consisting of C, N, O, S and P. Exemplary linkers include, but are not limited to, a substituted alkyl or a substituted cycloalkyl. Alternately, the heterologous moiety may be directly attached (where the linker is a single bond) to the amino or carboxy terminus of the cell-penetrating peptide. When the linker is not a single covalent bond, the linker may be any combination of stable chemical bonds, optionally including, single, double, triple or aromatic carbon-carbon bonds, as well as carbon-nitrogen bonds, nitrogen-nitrogen bonds, carbon-oxygen bonds, sulfur-sulfur bonds, carbon-sulfur bonds, phosphorus-oxygen bonds, phosphorus-nitrogen bonds, and nitrogen-platinum bonds. The linker may incorporate less than 20 nonhydrogen atoms and are composed of any combination of ether, thioether, urea, thiourea, amine, ester, carboxamide, sulfonamide, hydrazide bonds and aromatic or heteroaromatic bonds. The linker may be a combination of single carbon-carbon bonds and carboxamide, sulfonamide or thioether bonds.

A general strategy for conjugation involves preparing the cell-penetrating peptide and the apoptotic target inhibitor components separately, wherein each is modified or derivatized with appropriate reactive groups to allow for linkage between the two. The modified the apoptotic target inhibitor is then incubated together with a cell-penetrating peptide that is prepared for linkage, for a sufficient time (and under such appropriate conditions of temperature, pH, molar ratio, etc.) as to generate a covalent bond between the cell-penetrating peptide and the apoptotic target inhibitor molecule.

Numerous methods and strategies of conjugation will be readily apparent to one of ordinary skill in the art, as will the conditions required for efficient conjugation. By way of example only, one such strategy for conjugation is described below, although other techniques, such as the production of fusion proteins or the use of chemical linkers is within the scope of the instant invention.

When generating a disulfide bond between the apoptotic target inhibitor molecule and the cell-penetrating peptide of the present invention, the apoptotic target inhibitor molecule can be modified to contain a thiol group, and a nitropyridyl leaving group can be manufactured on a cysteine residue of the cell-penetrating peptide. Any suitable bond (e.g., thioester bonds, thioether bonds, carbamate bonds, etc.) can be created according to methods generally and well known in the art. Both the derivatized or modified cell-penetrating peptide, and the thiol-containing apoptotic target inhibitor are reconstituted in RNase/DNase sterile water, and then added to each other in amounts appropriate for conjugation (e.g., equimolar amounts). The conjugation mixture is then incubated for 15 min at 65°C, followed by 60 min at 37°C., and then stored at 4°C. Linkage can be checked by running the vector-linked apoptotic target inhibitor molecule, and an aliquot that has been reduced with DTT, on a 15% non-denaturing PAGE. Apoptotic target inhibitor molecules can then be visualized with the appropriate stain.

The AICPP will comprise a double stranded nucleic acid conjugated to a cell-penetrating peptide. At least one strand of the double-stranded ribonucleic acid molecule (either the sense or the antisense strand) may be modified for linkage with a cell-penetrating peptide (e.g., with a thiol group), so that the covalent bond links the modified strand to the cell-penetrating peptide. Where the strand is modified with a thiol group, the covalent bond linking the cell-penetrating peptide and the modified strand of the ribonucleic acid molecule can be a disulfide bond, as is the case where the cell-penetrating peptide has a free thiol function (i.e., pyridyl disulfide or a free cysteine residue) for coupling. However, it will be apparent to those skilled in the art that a wide variety of functional groups may be used in the modification of the ribonucleic acid, so that a wide variety of covalent bonds (e.g., ester bonds, carbamate bonds, sulfonate bonds, etc.) may be applicable. Additionally, the membrane-permeable complex of the present invention may further comprise a moiety conferring target-cell specificity to the complex. The present invention may be directed to a penetratin1- XBIR3 conjugate. The sequence of the penetratin1-XBIR3 sequence may be PEN1-XBIR3: RQIKIWFQNRRMKWKK-s-s-NTLPRNPSMADYEARIFTFGTWIYSVNKEQLARAGF YALGEGDKVKCFHCGGGLTDWRPSEDPWEQHARWYPGCRYLLEQRGQEYI NNIHLTHS. The disclosure relates to a conjugate of penetratin1 and a dominant negative form of a caspase polypeptide. The dominant negative form of caspase-6 may be the polypeptide designated "C6DN" in Denault, J.B. and G.S. Salvesen, Expression, purification, and characterization of caspases. Curr Protoc Protein Sci, 2003. Chapter 21: p. Unit 21 13, and the sequence of penetratin1-C6DN is

RQIKIWFQNRRMKWKK-s-s-
MASSASGLRRGHPAGGEENMTETDAFYKREMFDPAEKYKMDHRRRGIALIF
NHERFFWHLTLPERRGTCADRDNLTRRFSDLGFEVKCFNDLKAEELLLKIHEV
STVSHADADCFVCVFLSHGEGNHIYAYDAKIEIQTLTGLFKGDKCHSLVGKPK
IFIIQAARGNQHDVPVIPLDVVDNQTEKLDTNITEVDAASVYTLPAGADFLMC
YSVAEGYYSHRETVNGSWYIQDLCEMLGKYGSSLEFTELLTLVNRKVSQRRV
DFCKDPSAIGKKQVPCFASMLTKKLHFFPKSNLEHHHH

### 5.1.3 Pharmaceutical Compositions

The apoptotic target inhibitors or membrane-permeable complexes of the instant invention are formulated for nasal administration. For nasal administration, solutions or suspensions comprising the apoptotic target inhibitors or membrane-permeable complexes of the instant invention can be formulated for direct application to the nasal cavity by conventional means, for example with a dropper, pipette or spray. Other means for delivering the nasal spray composition, such as inhalation via a metered dose inhaler (MDI), may also be used according to the present invention. Several types of MDIs are regularly used for administration by inhalation. These types of devices can include breath-actuated MDI, dry powder inhaler (DPI), spacer/holding chambers in combination with MDI, and nebulizers. The term "MDI" as used herein refers to an inhalation delivery system comprising, for example, a canister containing an active agent dissolved or suspended in a propellant optionally with one or more excipients, a metered dose valve, an actuator, and a mouthpiece. The canister is usually filled with a solution or suspension of an active agent, such as the nasal spray composition, and a propellant, such as one or more hydrofluoroalkanes. When the actuator is depressed a metered dose of the solution is aerosolized for inhalation. Particles comprising the active agent are propelled toward the mouthpiece where they may then be inhaled by a subject. The formulations may be provided in single or multidose form. For example, in the case of a dropper or pipette, this may be achieved by the patient administering an appropriate, predetermined volume of the solution or suspension. In the case of a spray, this may be achieved for example by means of a metering atomising spray pump. To improve nasal delivery and retention the components according to the invention may be encapsulated with cyclodextrins, or formulated with agents expected to enhance delivery and retention in the nasal mucosa.

Commercially available administration devices that are used or can be adapted for nasal administration of a composition of the invention include the AERONEB™ (Aerogen, San Francisco, Calif), AERONEB GO™ (Aerogen); PARI LC PLUS™, PARI BOY™ N, PARI™ eflow (a nebulizer disclosed in U.S. Pat. No. 6,962,151), PARI LC SINUS™, PARI SINUSTAR™., PARI SINUNEB™, VibrENT™ and PARI DURANEB™ (PARI Respiratory Equipment, Inc., Monterey, Calif. or Munich, Germany); MICROAIR™ (Omron Healthcare, Inc, Vernon Hills, Ill.), HALOLITE™ (Profile Therapeutics Inc, Boston, Mass.), RESPIMAT™ (Boehringer Ingelheim, Germany), AERODOSE™ (Aerogen, Inc, Mountain View, Calif.), OMRON ELITE™ (Omron Healthcare, Inc, Vernon Hills, 111.), OMRON MICROAIR™ (Omron Healthcare, Inc, Vernon Hills, Ill.), MABISMIST™ II (Mabis Healthcare, Inc, Lake Forest, Ill.), LUMISCOPE™ 6610, (The Lumiscope Company, Inc, East Brunswick, N.J.), AIRSEP MYSTIQUE™, (AirSep Corporation, Buffalo, N.Y.), ACORN-1™ and ACORN-II™ (Vital Signs, Inc, Totowa, N.J.), AQUATOWER™ (Medical Industries America, Adel, Iowa), AVA-NEB™ (Hudson Respiratory Care Incorporated, Temecula, Calif.), AEROCURRENT™ utilizing the AEROCELL™ disposable cartridge (AerovectRx Corporation, Atlanta, Ga.), CIRRUS™ (Intersurgical Incorporated, Liverpool, N.Y.), DART™ (Professional Medical Products, Greenwood, S.C.), DEVILBISS™ PULMO AIDE (DeVilbiss Corp; Somerset, Pa.), DOWNDRAFT™ (Marquest, Englewood, Colo.), FAN JET™ (Marquest, Englewood, Colo.), MB-5™ (Mefar, Bovezzo, Italy), MISTY NEB™ (Baxter, Valencia, Calif.), SALTER 8900™ (Salter Labs, Arvin, Calif.), SIDESTREAM™ (Medic-Aid, Sussex, UK), UPDRAFT-II™ (Hudson Respiratory Care; Temecula, Calif.), WHISPER JET™ (Marquest Medical Products, Englewood, Colo.), AIOLOS™ (Aiolos Medicnnsk Teknik, Karlstad, Sweden), INSPIRON™ (Intertech Resources, Inc., Bannockburn, Ill.), OPTIMIST™ (Unomedical Inc., McAllen, Tex.), PRODOMO™, SPIRA™ (Respiratory Care Center, Hameenlinna, Finland), AERx™ Essence™ and Ultra™, (Aradigm Corporation, Hayward, Calif.), SONIK™ LDI Nebulizer (Evit Labs, Sacramento, Calif.), ACCUSPRAY™ (BD Medical, Franklin Lake, N.J.), ViaNase ID™ (electronic atomizer; Kurve, Bothell, Wash.), OptiMist™ device or OPTINOSE™ (Oslo, Norway), MAD Nasal™ (Wolfe Tory Medical, Inc., Salt Lake City, Utah), Freepod™ (Valois, Marly le Roi, France), Dolphin™ (Valois), Monopowder™ (Valois), Equadel™ (Valois), VP3™ and VP7™ (Valois), VP6 Pump™ (Valois), Standard Systems Pumps™ (Ing. Erich Pfeiffer, Radolfzell, Germany), AmPump™ (Ing. Erich Pfeiffer), Counting Pump™ (Ing. Erich Pfeiffer), Advanced Preservative Free System™ (Ing. Erich Pfeiffer), Unit Dose System™ (Ing. Erich Pfeiffer), Bidose System™ (Ing. Erich Pfeiffer), Bidose Powder System™ (Ing. Erich Pfeiffer), Sinus Science™ (Aerosol Science Laboratories, Inc., Camarillo, Calif.), ChiSys™ (Archimedes, Reading, UK), Fit-Lizer™ (Bioactis, Ltd, a SNBL subsidiary (Tokyo, J P), Swordfish V™ (Mystic Pharmaceuticals, Austin, Tex.), DirectHaler™ Nasal (DirectHaler, Copenhagen, Denmark) and SWIRLER™ Radioaerosol System (AMICI, Inc., Spring City, Pa.).

To facilitate delivery to a cell, tissue, or subject, the apoptotic target inhibitor or membrane-permeable complex of the present invention may, in various compositions, be formulated with a pharmaceutically-acceptable carrier, excipient, or diluent. The term "pharmaceutically-acceptable", as used herein, means that the carrier, excipient, or diluent of choice does not adversely affect either the biological activity of the apoptotic target inhibitor or membrane-permeable complex or the biological activity of the recipient of the composition. Suitable pharmaceutical carriers, excipients, and/or diluents for use in the present invention include, but are not limited to, lactose, sucrose, starch powder, talc powder, cellulose esters of alkonoic acids, magnesium stearate, magnesium oxide, crystalline cellulose, methyl cellulose, carboxymethyl cellulose, gelatin, glycerin, sodium alginate, gum arabic, acacia gum, sodium and calcium salts of phosphoric and sulfuric acids, polyvinylpyrrolidone and/or polyvinyl alcohol, saline, and water. Specific formulations of compounds for therapeutic treatment are discussed in Hoover, J. E., Remington's Pharmaceutical Sciences (Easton, Pa.: Mack Publishing Co., 1975) and Liberman and Lachman, eds., Pharmaceutical Dosage Forms (New York, N.Y.: Marcel Decker Publishers, 1980).

In accordance with the methods disclosed herein, the quantity of the apoptotic target inhibitor or membrane-permeable complex that is administered to a cell, tissue, or subject should be an amount that is effective to inhibit the apoptotic target within the tissue or subject. This amount is readily determined by the practitioner skilled in the art. The specific dosage employed will depend upon a number of factors, including the type inhibitor used, the apoptotic target to be inhibited, and the cell type expressing the target. Quantities will be adjusted for the body weight of the subject, and the particular disease or condition being targeted.

### 5.2 Methods of Treatment

The disclosure relates to methods of ameliorating the impact of CNS ischemic injury or decreasing the risk or manifestation of neurodegenerative disease. For example, methods of administering an effective amount of an AICPP conjugate in order to inhibit apoptosis associated with ischemic injury and thereby ameliorate the impact of the ischemic injury.

The methods may be directed to the intranasal administration of an apoptotic target inhibitor in order to inhibit apoptosis associated with ischemic injury in the central nervous system. The AICPP conjugate may be administered during a treatment window that begins at the onset of ischemia and extends over the next 48 hours, where treatment is preferably administered within about 24 hours or within about 12 hours of the ischemic event. The disclosure relates to methods for ameliorating the impact of ischemic injury that can be practiced beyond the traditional window for treatments (e.g., treatment with tissue plasminogin activator (tPA) must generally be administered within 3 hours of onset of ischemic injury). The methods may be used to to treat a patient who has experienced a sudden onset of a neurological deficit that would be consistent with a diagnosis of cerebral infarction or transient ischemic attack; for example, such neurologic deficit may be an impairment of speech, sensation, or motor function.

The treatment, when used to either treat/ameliorate the effects of ischemia or treat neurodegenerative disease, may be administered as a single dose or multiple doses; where multiple doses are administered, they may be administered at intervals of 6 times per 24 hours or 4 times per 24 hours or 3 times per 24 hours or 2 times per 24 hours. The initial dose may be greater than subsequent doses or all doses may be the same.

A polypeptide apoptotic target inhibitor, such as, but not limited to Pen1-XBIR3 or a dominant negative form of a caspase may be employed to treat ischemia. A Pen1-XBIR3 or dominant negative form of a caspase AICPP conjugate may be administered to a patient suffering from an ischemic injury either as a single dose or in multiple doses. Where multiple doses are administered, they may be administered at intervals of 6 times per 24 hours or 4 times per 24 hours or 3 times per 24 hours or 2 times per 24 hours. The initial dose may be greater than subsequent doses or all doses may be the same. The concentration of the Pen1-XBIR3 or dominant negative form of a caspase AICPP composition administered may be 0.01µM to 1000µM; 1 µM to 500 µM; or 10 µM to 100 µM). The Pen1-XBIR3 or dominant negative form of a caspase AICPP composition may be delivered nasally by administering drops of 0.1µl to 1000µl; 1.0µl to 500µl; or 10µl to 100µl to alternating nares every 30 seconds to five minutes; every one minute to every four minutes; or every two minutes for 10 to 60 minutes; every 15 to 30 minutes; or every 20 minutes. A specific human equivalent dosage can be calculated from animal studies via body surface area comparisons, as outlined in Reagan-Shaw et al., FASEB J., 22; 659-661 (2007).

Pen1-XBIR3 or dominant negative form of a caspase may be employed to treat neurodegenerative disease. A Pen1-XBIR3 or dominant negative form of a caspase AICPP conjugate may be administered to a patient suffering from a neurodegenerative disease either as a single dose or in multiple doses. Where multiple doses are administered, they may be administered at intervals of 6 times per 24 hours or 4 times per 24 hours or 3 times per 24 hours or 2 times per 24 hours. The initial dose may be greater than subsequent doses or all doses may be the same. The concentration of the Pen1-XBIR3 or dominant negative form of a

caspase AICPP composition administered is, in certain embodiments: 0.01µM to 1000µM; 1 µM to 500 µM; or 10 µM to 100 µM). The Pen1-XBIR3 or dominant negative form of a caspase AICPP composition may be delivered nasally by administering drops of 0.1µl to 1000µl; 1.0µl to 500µl; or 10µl to 100µl to alternating nares every 30 seconds to five minutes; every one minute to every four minutes; or every two minutes for 10 to 60 minutes; every 15 to 30 minutes; or every 20 minutes. A specific human equivalent dosage can be calculated from animal studies via body surface area comparisons, as outlined in Reagan-Shaw et al., FASEB J., 22; 659-661 (2007).

The apoptotic target inhibitor, either alone or in the context of a membrane-permeable complex may be administered in conjunction with one or more additional therapeutics. The additional therapeutics may include, but are not limited to, anticoagulant agents, such as tPA or heparin, free radical scavengers, anti-glutamate agents, etc. (see, for example, Zaleska et al., 2009, Neuropharmacol. 56(2):329-341). The method may involve the administration of one or more additional apoptotic target inhibitors either alone or in the context of a membrane-permeable complex.

### 5.3 Medical uses

There is provided a compound for use in the treatment of ischemic injury in the central nervous system, wherein the compound is administered intranasally and comprises a caspase inhibitor covalently conjugated to a cell-penetrating peptide, wherein said caspase inhibitor is selected from the group consisting of: (i) a caspase-6 dominant negative (C6DN), (ii) XBIR3, and (iii) a peptide that is capable of apoptotic target inhibition and has at least 70% homology with either a C6DN or a XBIR3; and said cell-penetrating peptide is selected from the group consisting of penetratin-1, transportan, pIS1, Tat(48-60), pVEC, MAP, and MTS. The cell-penetrating peptide may be penetratin-1. The peptide caspase inhibitor may be C6DN. The peptide caspase inhibitor may be XBIR3. The caspase inhibitor may be administered during a window of time in which the inhibitor target is either expressed or active. The caspase inhibitor is XBIR3 and the administration may occur between the onset of the ischemic injury and 24 hours post reperfusion. The caspase inhibitor may be Penl-C6DN and the administration may occur between 12 and 24 hours post reperfusion. The peptide caspase inhibitor may be C6DN and the cell penetrating peptide may be penetratin-1. The peptide caspase inhibitor may be selected from the group consisting of (i) a disulfide-linked Penetratin1-C6DN, and (ii) a disulfide-linked Penetratin1-XBIR3.

### 6. EXAMPLES

### 6.1 Caspase-6 in Axon Loss and Neurodegeneration

The instant examples establish that caspase-6 is a mediator of axonal degeneration and neuronal loss following cerebral ischemia and that inhibition of caspase-6 activity is neuroprotective in vivo. As outlined in section 6.2, below, active caspase-6 is temporally induced in cell bodies and neuronal processes following ischemia in both rats and mice. Genetic knockout of caspase-6 is shown in section 6.3 to be neuroprotective against stroke and ameliorates neurofunctional deficits associated with stroke. Furthermore, the time course of caspase-6 activation corresponds with that of axonal degeneration observed in human stroke as well as other rodent models and this activation of caspase-6 in axons and dendrites by 12-24hpr makes it an attractive molecular target for neuroprotection. As outlined in section 6.4, below, an in vitro technique for trapping active caspases (Tu, S. et al., Nat Cell Biol 8 (1), 72-77 (2006)) for use in vivo has been employed and it is found that caspase-9 is active at 1hpr and 4hpr. (Akpan et al., J. Neuroscience 31 (24), 8894-8904 (2011)). To determine whether caspase-9 activation leads to caspase-6 cleavage, caspase-9 activity was inhibited with the BIR3 domain from XIAP (XBIR3), a member of the Inhibitor of Apoptosis family of proteins (see section 6.5). This protein domain, a highly specific inhibitor of caspase-9, was linked to Penetratin1 (Pen1), a cell transduction peptide, in order to deliver it across the plasma membrane. (Eckelman, et al., EMBO Rep 7 (10), 988-994 (2006)). Intraparenchymal convection enhanced delivery strategy as well intranasal delivery of Pen1-XBrR3 inhibits caspase-6 activation in neuronal processes and is neuroprotective. Furthermore, as outlined in section 6.6, intranasal delivery of Pen1-XBTR3 provides functional neuroprotection in vivo. In summary, these examples establish that caspase-6 and caspase-9 are active in axon degeneration and neuron death in stroke and their inhibition can ameliorate the impact of ischemic injury

### 6.2 Caspase-6 is Active in Neuronal Processes and Soma Following Stroke

Many caspases are implicated in the progression of neurodegeneration in stroke, but clear evidence for the specific role of individual caspases remains elusive. (Ribe, et al., Biochem J 415 (2), 165-182 (2008)). The instant example examines whether caspase-6 was activated in neuronal processes after in vivo ischemia. Rats were subjected to 2 hours of transient middle cerebral artery occlusion (tMCAo) and brains were imaged for cleaved caspase-6 (cl-C6) at increasing times post-reperfusion. Because cleavage of caspase-6 between the large and small subunits fully activates this protease, antiserum-reactivity to the neo-epitope generated by cleavage is an authentic readout of activation. (Stennicke, et al., Methods Enzym. 17 (4), 313-319 (1999)). The penumbral region in the forebrain, specifically cortical layers I-IV in the granular insular, somatosensory, dorsal motor cortices (Fig. la), revealed a temporal increase in staining for cl-C6 (Fig. 1b). No cl-C6 was detected in control non-ischemic animals. By 4hpr there was minimal staining in the penumbra, but by 12hpr there was abundant cl-C6 staining in processes and cell bodies in the cingulate, primary motor, primary and secondary somatosensory, and granular insular cortices (Fig. 1a,b). There was progressive activation of C6 in the nuclei by 24hpr, which continued through 3 days post reperfusion (dpr). At 7dpr cl-C6 was only seen in nuclei. In wild-type mice subjected to tMCAo, the pattern of staining was similar, with cell body and process staining detected at 24hpr and 3dpr (Fig, 1c). Neurologically this time course corresponds both to the progression of the infarct, with expansion of the infarct over the first 3 days, and with axon degeneration. Costaining with NeuN showed cl-C6 was located in neurons (Fig. Id), whereas there was no colocalization with GFAP, a marker for astrocytes. In order to identify whether cl-C6 was present in axons or dendrites, sections were co-stained for cl-C6 and Tuj1 or NF-L (axon markers) or MAP-2 (dendrite marker). At 24hpr, Tuj1 and cl-C6 were found in single neuron processes (Fig. 1e). These processes were not continuous and gaps in the process were positive for cl-C6. Interestingly, previous work in AD suggests caspase-6 cleaves tubulin and tau, which may disrupt microtubule and axon stability. (Klaiman, et al., Mol Cell Proteomics 7 (8), 1541-1555 (2008); Guo, et al., Am J Pathol 165 (2), 523-531 (2004)). C1-C6 is also found in single processes containing NF-L or MAP-2 (Fig. 1e), with similar cl-C6 filled gaps in the process staining. Such function can be the result of caspase-6 is directly cleaving these proteins or associated proteins that stabilize their polymerization.

### 6.3 Genetic Knockout of Caspase-6 is Neuroprotective

Caspase null mice ("caspase-6^{-/-}") are powerful instruments for studying the role of these proteases in cerebral ischemia. Wild-type and caspase-6^{-/-} mice were subjected to tMCAo, and caspase-6^{-/-} mice (Fig. 2a) showed significantly better neurological function at 24hpr compared to wild-type mice based on a 28-point exam (Fig. 2b, and 2c). (Clark, et al., Neurol Res 19 (6), 641-648 (1997)). Similar neuroprotection was previously observed in caspase-3 null mice subjected to tMCAo. (Le, et al., Proc Natl Acad Sci USA 99 (23), 15188-15193 (2002)). 2,3,5-Triphenyltetrazolium chloride (TTC) staining, a common measure of infarct volume, showed no significant difference at 24hpr, despite the significant difference in neurofunction. To study this further, neuronal and process number were quantified. Wild-type mice subjected to 1hr tMCAo followed by 24hpr showed a 47% decrease in neuronal number compared to non-stroked wildtype mice, this decrease was partially rescued in caspase-6^{-/-} mice (Fig. 2d). Fluorescent nissl (NeuroTrace) staining yielded similar results. This indicated that cell counting and neurofunction exam provide more sensitive measures than TTC at this time point. Additionally, wild-type mice subjected to tMCAo had fewer NF-L-positive processes compared to caspase-6^{-/-} mice (Fig. 2e). Processes from wild-type mice were shorter and exhibited more fragmented NF-L staining, suggestive of axon fragmentation and degeneration. There were also fewer processes with MAP-2 in stroked wild-type mice compared to caspase-6^{-/-} (Fig. 2e). Tau is a putative axonal substrate for caspase-6 with potential cleavage sites in N-terminal and C-terminal regions of tau. (Guo, et al., Am J Pathol 165 (2), 523-531 (2004); Horowitz, et al., J Neurosci 24 (36), 7895-7902 (2004)). Analysis with an antibody specific to the C-terminal region of tau revealed that caspase-6^{-/-} brain retained more intact tau than wild-type brain at 24hpr (Fig. 2F). This suggests that caspase-6 reduces tau levels during stroke. This loss of tau can lead to microtubule instability and loss of process integrity.

### 6.4 Caspase-9, an Initiator Caspase, is Active Early in Stroke

Caspase-6 is an effector caspase, and prior work showed that the initiator caspase, caspase-9, leads to the activation of caspase-6. (Pop & Salvesen, J Biol Chem 284 (33), 21777-21781 (2009)). The induction of detectable cleaved caspase-6 by 12hpr suggested that initiator caspase activation must occur prior to this time point. While activation of effector caspases requires cleavage, allowing the use of cleavage specific antibodies to determine the activation state, initiator caspases do not require cleavage for activation, but can be activated by dimerization. (Ribe, et al., Biochem J 415 (2), 165-182 (2008)). At present the caspase activity based probe biotin-VAD-fmk (bVAD) is the best way to determine if initiator caspases are active after a death stimulus. bVAD is an irreversible pan-caspase inhibitor that has been used in vitro to identify caspase activation following various death stimuli. (Tu, et al., Nat Cell Biol 8 (1), 72-77 (2006); Denault & Salvesen, J Biol Chem 278 (36), 34042-34050 (2003); Tizon, et al., J Alzheimers Dis 19 (3), 885-94 (2009)). bVAD will irreversibly bind to any active caspase and inhibit downstream events. Eventually initiator caspases are cleaved, but this is a downstream consequence of their activation. (Malladi, et al., EMBO J 28 (13), 1916-1925 (2009); Denault & Salvesen, Methods Mol Biol 414, 191-220 (2008); Srinivasula, et al., Nature 410 (6824), 112-116 (2001)). This method has been adapted for use in cultured primary neurons and now it has been further adapted for use in vivo in the CNS. (Tizon, et al., J Alzheimers Dis 19 (3), 885-94 (2009)). To determine whether initiator caspases were activated early in stroke, rats were injected with 200nmoles bVAD via convection enhanced delivery to the striatum 1hr prior to tMCAo and sacrificed at 1hpr. The injected region was dissected, and bVAD-caspase complexes were isolated on streptavidin-agarose beads and analyzed by western blotting. bVAD captured caspase-9 (Fig. 3a) and caspase-8, showing activation of these initiator caspases is an early event in stroke. Caspases-1 and -2 were not isolated by bVAD. To determine if caspase-9 continues to be activated, animals were treated as in 3a and sacrificed at 4hpr. bVAD captured caspase-9 (Fig. 3b), showing that caspase-9 continues to be activated as the stroke progresses. Additionally, at 24hpr it was observed that cells positive for cl-C6 were also positive for caspase-9 (Fig. 3c). Caspase-9 was observed in processes along with cl-C6. Based on these data, it is considered that caspase-9 can regulate caspase-6 activity and thus this relationship was explored further.

### 6.5 Caspase-9 Activates Caspase-6 in Processes and Soma of Neurons

The co-localization of caspase-9 and cl-C6 supports a mechanism for caspase-9 activating caspase-6. To determine if caspase-9 was activating caspase-6, testing was undertaken to investigate whether inhibition of caspase-9 would block caspase-6 activation. Currently available small molecule inhibitors are not sufficiently specific to dissect the contribution of individual caspases, so an alternative approach to explicitly inhibit caspase-9 was developed. (McStay, et al., Cell Death Differ 15 (2), 322-331 (2008)). Mammals express a family of cell death inhibiting proteins known as IAPs. One member of this family, X-linked IAP or (XIAP), is a potent, specific inhibitor of active caspases-9, -3, -7. IAPs contain baculoviral IAP repeat (BIR) domains, and for XIAP caspase inhibition specificity is dependent on specific BIR domains, with the BIR3 domain specifically targeting active caspase-9. (Eckelman, et al., EMBO Rep 7 (10), 988-994 (2006)).

To facilitate intracellular uptake of XIAP-BIR3 the peptide was disulfide-linked to Penetratin1, a cell transduction peptide. (Davidson, et al., J Neurosci 24 (45), 10040-10046 (2004)). Upon entry into the cell the disulfide linkage is broken by the reducing environment of the cytoplasm, releasing the peptide cargo and allowing it to act at its target. Functional efficacy of this construct was confirmed using hippocampal neuronal cultures that were subjected to 4-hydroxynonenal (HNE) mediated death, a caspase-9 dependent death. (Rabacchi, et al., Neurobiol Aging 25 (8), 1057-1066 (2004)). Treatment of cultures with Penl-XBIR3 and HNE abrogated death. To ensure that a Penl-peptide could be delivered to the brain, Penl was linked to a FITC-labeled control peptide and delivered to the striatum using convection enhanced delivery (CED). Brains were harvested 24hr after delivery, sectioned, and imaged. The FITC-peptide was distributed throughout the ipsilateral hemisphere, and the higher power image revealed intracellular uptake. Pen1-XBIR3 was delivered to the striatum 1hr prior to tMCAo using ICC. Animals were harvested at 24hpr and immunostained for caspase-9 and cl-C6. Pen1-XBIR3 inhibited appearance of cl-C6 and caspase-9 in cell bodies and processes (Fig. 3c). Thus, caspase-9 activity was necessary for activation of caspase-6 in neuron soma and processes following a transient ischemic event in rats.

The preceding findings demonstrate that intraparenchymal delivery of Pen1-XBIR3 prevents activation of caspase-6. The following experiment was performed to ascertain if the Penl-XBIR3 could also bypass the blood brain barrier via another delivery technique. Intranasal delivery of neurotrophins and other compounds has been demonstrated to provide access to the CNS to prevent neurodegeneration in a number of models including stroke. (Dhuria, et al., J Pharm Sci 99 (4), 1654-1673 (2010); Liu, et al., J Stroke Cerebrovasc Dis 13 (1), 16-23 (2004); Liu, et al., J Neurol Sci 187 (1-2), 91-97 (2001)). This delivery method takes advantage of the olfactory pathway to bypass the blood brain barrier, however until now, proteins and compounds delivered via this method in rodent models have targeted extracellular targets, such as cell surface receptors. Since caspases, which are intracellular proteins, are targeted in this experiment, the cargo needed to be delivered intracellularly. As shown above, Penetratin1 provides the necessary intracellular uptake of linked peptides. Penl-XBIR3 was delivered intranasally to rats, after which brains were sliced coronally, and the presence of XBIR3 in the CNS determined by western blotting (Fig. 4a). Pen1-XBIR3 was delivered to all slices of the brain, similar to the delivery pattern for IGF41.

To determine if intranasal delivery of Pen1-XBIR3 also reduced caspase-6 activity, axon/dendrite loss and provided neuroprotection from stroke, animals were treated with Pen1-XBIR3 1hr prior to tMCAo and harvested at the indicated times of reperfusion. Brains were analyzed for expression of activated caspase-6, NF-L, MAP-2, and NeuN at 12hpr and 24hpr. While Pen1-XBIR3 did not significantly reduce caspase-6 activation in processes by 12hpr, there was a trend towards a decrease at this time point (Fig. 4b). By 24hpr, there was a significant reduction of cl-C6 in processes by 24hpr (Fig. 4b) compared to rats treated with saline. Therefore, caspase-9 inhibition using this delivery technique reduced caspase-6 activity. Moreover, at 24hpr Penl-XBIR3 provided significant protection against neuron loss; there is no apparent neuron loss in any of the groups at 12hpr (Fig. 4b). In contrast to neuron density, the number of NF-L positive neurites was significantly decreased at 12hpr, suggestive of axon loss occurring prior to neuronal soma loss (Fig. 4c). This suggests that axon degeneration precedes neuron death in stroke, which has been proposed previously for other neurodegenerative diseases. (Coleman, M., Nat Rev Neurosci 6 (11), 889-898 (2005)). Axon protection by intranasal Penl-XBIR3 continued through 24hpr (Fig. 4c). Unlike axon density, dendrite levels are unaffected at 12 and 24hpr (Fig. 4d), which can indicate a slower time-course for dendritic degeneration or a different mechanism of degeneration.

To determine if caspase-6 is active in human stroke, post-mortem tissue from brains of patients who had died following ischemic stroke was immunostained for cl-C6. DAB developing (Fig. 5a) showed staining of cell bodies and processes in the infarcted tissue; NF-L staining of adjacent sections showed a decrease in process density. To determine if cl-C6 colocalized with a marker for processes, sections were co-stained for cl-C6 and Tuj 1 (Fig. 5b). C1-C6 was found in a process in the ischemic tissue, and the pattern of co-localization with Tuj 1 was very similar to that observed in the rodent models of ischemia

### 6.6 Intranasal Penl-XBIR3 Provides Functional Neuroprotection In Vivo

The efficacy of Pen1-XBIR3 to prevent sensory-motor disability caused by stroke was tested by giving rats either a prophylatic (pre-occlusion) or therapeutic (4 hours post reperfusion) intranasal bolus of vehicle or Penl-XBIR3 (prepared and administerd as described in section 6.8, below). Rodents were assayed with a 24-point neurofunctional scale starting at 1 day post-ischemia with testing every other day for 3 weeks after the ischemic event. Animals treated with Penl-XBIR3, prophylatically or 4 hours post reperfusion, exhibited less stroke related disability than their vehicle treated counterparts (Figure 6). Therapeutic protection by Pen1-XBIR3 indicates that caspase-9 activation is persistent at least up to 4 hours post reperfusion during stroke, as shown in Fig. 3b, and that this pathway is critical for the acute neurodegeneration elicited by stroke.

### 6.7 Intranasal Penl-C6DN Prevents Cleavage of Caspase-6 Substrates

To determine if a direct blockade of caspase-6 would provide protection from ischemia, a Penl- linked caspase-6 domininat negative (Pen1-C6DN) construct was utilized. Penl-C6DN was delivered by intransasal bolus to mice 1hr prior to tMCAo and mice were then subjected to 1hrMCAo followed by reperfusion. Animals were sacrificed at 24hpr and core and penumbra regions of brain prepared for Western blotting. As depicted in Figure 7, protein lysate from the core and penumbra regions of the stroke infarct (24hpr) was isolated. Ipsilateral (stroked) hemispheres contained abundant caspase-cleaved tau when only treated with vehicle. Penl-C6DN reduced cleavage of caspase-cleaved tau indicating that intranasal Penl-C6DN can prevent cleavage of caspase-6 substrates during stroke.

### 6.8 Data Analysis

These data show that caspases-6 and -9 are regulators of axon degeneration and neuron loss in cerebral ischemia. Figure 8 provides a schematic indicating activation of caspase-9 and -6 in ischemia and the effects of intervention in this activation. Caspase-6 is activated in the penumbral region in neuronal processes and cell bodies in both rat and mouse models as well as in human peri-infarct tissue. Genetic ablation of caspase-6 provides neuroprotection at the structural and functional levels. Functions for caspase-6 in neurons include processing huntingtin, which is associated with neurodegeneration in Huntington's disease. (Graham, et al., Cell 125 (6), 1179-1191 (2006)). Caspase-6 can cleave tau, affecting its ability to stabilize microtubules, and caspase-6-mediated cleavage of tau may play a role in AD pathogenesis. (Horowitz, et al., J Neurosci 24 (36), 7895-7902 (2004); Klaiman, et al., Mol Cell Proteomics 7 (8), 1541-1555 (2008); Guo, et al., Am J Pathol 165 (2), 523-531 (2004)). In the above-described models of cerebral ischemia, see sections 6.1-6.5, active caspase-6 co-localized with axonal and dendritic markers, implicating this caspase in the degeneration of neuronal processes. Although present in the same process, some areas with active caspase-6 lacked the process marker, suggesting that caspase-6 was cleaving the marker. In support of this function for caspase 6 in stroke, a reduction in tau in wild-type mice subjected to tMCAo relative to caspase-6^{-/-} mice was observed. Intranasal delivery of Penl-C6DN, a caspase-6 inhibitor, reduced the appearance of caspase-cleaved tau, indicating that targeting caspase-6 in stroke will provide functional neuroprotection. Further proteomic analysis of tissue lysate from infarcted tissue from caspase-6^{-/-} and wild-type mice can be used to reveal a broader spectrum of proteins cleaved by caspase-6 during stroke, and potentially many that regulate axon stability.

Moreover, caspase-6 is involved in process degeneration in dissociated DRG neurons subjected to trophic factor deprivation (Nikolaev, et al., Nature 457 (7232), 981-989 (2009)); that study proposed that caspase-6 is responsible for only process degeneration, but not for neuronal death. The instant studies find that caspase-6 is mediating both process degeneration and neuronal death during ischemia. The temporal activation of caspase-6 in the stroke penumbra corresponds with the progression of axonal degeneration. For other forms of neurodegeneration, axon degeneration is a major contributor to cell death and may instigate death via removal of target-derived trophic factors. (Ferri, et al., Curr Biol 13 (8), 669-673 (2003); Fischer, et al., Exp Neurol 185 (2), 232-240 (2004); Stokin, et al., Science 307 (5713), 1282-1288 (2005)). In these instances, axon degeneration preceded cell death. In clinical cases of cerebral ischemia, axon degeneration is observed as early as 2 days post ischemia (Thomalla, et al., Neuroimage 22 (4), 1767-1774 (2004)); however, the molecular events triggering axon degeneration may begin earlier. In the penumbral region, it is found that axon loss preceded neuronal loss, which indirectly suggests that axon degeneration precedes neuronal loss following an ischemic event.

Caspase-6 is an effector caspase that is activated by caspase-9. (Pop & Salvesen, J Biol Chem 284 (33), 21777-21781 (2009)). It is common practice to use short peptide caspase substrates for assaying caspase activity, however, these peptides are highly promiscuous and as such can generate misleading data. (McStay, et al., Cell Death Differ 15 (2), 322-331 (2008)). Biotin-VAD-fmk, an irreversible pan-caspase inhibitor, provides a reliable measurement of caspase activity through biochemical pulldown of active caspase complexes. Originally used to assay caspase activity in cell lines, and, more recently, in primary neuron cultures, this procedure has been adapted for in vivo use in the CNS. (Tu, et al., Nat Cell Biol 8 (1), 72-77 (2006); Tizon, et al., J Alzheimers Dis 19 (3), 885-94 (2009)). In the present study, it is demonstrated that caspase-9 is active in the core region early in the progression of the infarct (1 and 4hpr) by isolating active caspase-9 complexes with biotin-VADfmk.

There are a few putative mechanisms for how caspase-9 is activated in stroke and leads to caspase-6 cleavage. First, reactive oxygen species generated by hypoxia can result in DNA damage and the activation of p53. (Niizuma, et al., J Neurochem 109 Suppl 1, 133-138 (2009)). During apoptosis, activated p53 translocates to the mitochondrial outer membrane where it recruits Bcl-2 associated X protein (Bax) and other proapoptotic proteins. This recruitment leads to permeabilization of the outer mitochondrial membrane and releases cytochrome c into the cytosol, which leads to the activation of caspase-9. Alternatively, activation of caspase-9 and the resulting caspase-6 activation in ischemia can be receptor mediated. Both p75-neurotrophin receptor (p75NTR) and death receptor 6 (DR6) stimulation result in caspase-6 activation, and with DR6, axon degeneration. (Troy, et al., J Biol Chem 277 (37), 34295-34302 (2002); Nikolaev, et al., Nature 457 (7232), 981-989 (2009)). One of the many downstream targets of p75NTR is p53, which can lead to caspase-6 activation. One the interacting partners of DR6 is the tumor necrosis factor receptor type 1-associated death domain (TRADD), which binding to signal transducer TRAF2 and activates NF-kappaB. In relation to cell death function, NF-kappaB has both pro-apoptotic and anti-apoptotic function, but persistent activation of NF-kappaB in stroke is thought to be associated with driving a proapoptotic fate. (Ridder & Schwaninger, Neuroscience 158 (3), 995-1006 (2009). NF-kappaB regulates Bcl-2 family members (Bim, Bid, Bax, Bak) to effect mitochondrial membrane stability, cytochrome c release, and subsequently caspase-9 activation. (Ridder & Schwaninger, Neuroscience 158 (3), 995-1006 (2009))

As caspase-9 activity is stimulated early in stroke and elevated caspase-9 is observed in cells with cl-C6, caspase-9 is considered to lead to caspase-6 activation during stroke. The BIR3 domain from XIAP (a highly specific inhibitor of caspase-9) linked to Penetratinl (Penl), a transduction peptide that efficiently delivers cargo to cells was used to inhibit caspase-9 activity. (Davidson, et al., J Neurosci 24 (45), 10040-10046 (2004); Guegan, et al., Neurobiol Dis 22 (1), 177-186 (2006); Fan, et al., Neurochem Int 48 (1), 50-59 (2006)). Prior studies showed that intraperitoneal delivery of a fusion protein of PTDXBIR3-Ring reduces infarct volume following tMCAo. (Tu, et al., Nat Cell Biol 8 (1), 72-77 (2006); Guegan, et al., Neurobiol Dis 22 (1), 177-186 (2006); Fan, et al., Neurochem Int 48 (1), 50-59 (2006)). In the above-described studies, two different delivery strategies were employed to deliver this inhibitor to the brain. Convection enhanced delivery (CED) provides direct delivery to the region of the infarct; CED of this inhibitor prior to stroke abrogated the activation of caspase-6 in neuronal soma and processes. Therefore, caspase-9 activity regulates caspase-6 activity in stroke. From a therapeutic perspective, for CNS disorders, intranasal delivery is a very attractive treatment strategy as it provides direct access to the brain. This delivery combined with the cell permeant peptide Penetratin1 provides intracellular delivery to the CNS. The use of a disulfide linkage between Pen1 and the cargo peptide ensures that the cargo peptide can be functional once it is transported into the cell and released from Penl. In the present study, intranasal delivery of Pen1-XBIR3 inhibited caspase-6 activation, reduced axon degeneration and was neuroprotective. Although XBIR3 provides indirect caspase-6 inhibition by blocking caspase-9, the recent publication of the crystal structure of caspase-6 should lead to the generation of a more specific caspase-6 inhibitor. (Baumgartner, et al., Biochem J 423 (3), 429-439 (2009)). Furthermore, the data presented using Penl-C6DN indicates that this method provides direct inhibition of caspase-6. The instant data reveal that caspase-6 activation corresponds to axon degeneration in stroke, and provide insight into how this process occurs in ischemia. Since caspase-6 activation is relatively delayed following ischemic onset, efficacious inhibition of caspase-6 in stroke can provide substantial post-ischemic functional neuroprotection and a valuable therapeutic strategy for cerebral ischemia.

### 6.8 Materials and Methods

**Antibodies.** For immunohistochemistry, anti-Tuj 1 antibody (abcam ab7751), anti-neurofilament-L (Cell Signaling #2835), anti-MAP-2 (Sigma #M9942), anti-GFAP (Thermo Scientific PA1-10004), anti-full-length and cleaved caspase-9 (abcam ab28131; also used for western blotting), anti-cleaved caspase-6 (Cell Signaling #9761), anti-cleaved caspase-3 (Cell Signaling #9661), and anti-cleaved caspase-7 antibody (MBL #BV-3147-3). For Western blotting, THE™ anti-His (GenScript #A00186), anti-caspase-8 (abcam ab52183), anti-caspase-6 (BD #556581), Tau V-20 (Santa Cruz # sc-1996), Lamin A/C (MBL International #JM-3267-100).

**Mouse & Rat Stroke Models.** Caspase-6 null (C6^{-/-}) mice (Jackson Laboratories)^{48,49} on C57/B16 background were bred with wild-type C57/B16 mice to generate C6^{+/-} heterozygotes, hets were bred to generate C6^{-/-} and wild-type littermates for studies. 2-3 month old male C6^{-/-} and wild-type littermate mice (23-30g) as well as adult Wistar male rats 250-300g (Taconic Laboratories) were subjected to transient middle cerebral artery occlusion (tMCAo) as previously published. (Connolly, et al., Neurosurgery 38 (3), 523-531; discussion 532 (1996); Komotar, et al., Nat Protoc 2 (10), 2345-2347 (2007)). Brains were harvested and processed for western blotting or immunohistochemistry as described below. For mouse neurofunctional analysis, a 28 point neurological functional exam was performed as previously described. (Clark, et al., Neurol Res 19 (6), 641-648 (1997)). Additionally, single mice were placed in a fresh cage at each time point (Pre-stroke, 24hr reperfusion, 7 days reperfusion) short videos (3 min at each time point) were recorded of each mouse's representative spontaneous activity to illustrate motor deficits in the mouse stroke model.

**Convection enhanced delivery (CED) of biotin-VAD-fmk or Penl-XBIR3.** Adult male Wistar rats (250-300g) were anesthetized using isoflurane (2%) delivered via an anesthesia mask for stereotactic instruments (Stoelting) and positioned in a stereotactic frame. CED was performed as previously described with the following stereotactic coordinates (1 mm anterior, 3 mm lateral, 5 mm depth). (Bruce, et al., Neurosurgery 46 (3), 683-691 (2000)). Infusion of the therapeutic was then instituted at a rate of 0.5µl/ minute. Following infusion, the cannula was removed at a rate of 1mm/minute, the burrhole was sealed with bonewax, and the skin incision was closed with skin adhesive. Postprocedure, rats were placed in a 37°C post-operation incubator and maintained at normothermia for an hour.

**Pen1-XBIR3.** The BIR3 domain from XIAP (XBIR3) was purified as previously described. (Sun, et al., J Biol Chem 275 (43), 33777-33781 (2000)). Penetratin1 (Penl, Q-Biogene, Carlsbad, CA) was mixed at an equimolar ratio with purified XBIR3 and incubated overnight at 37°C to generate disulfide-linked Pen/BIR3. Linkage was assessed by 20% SDS-PAGE and western blotting with anti-His antibody. 30µl of Pen1-XBIR3 (36.8µM) was infused by ICC immediately prior to induction of ischemia. Animals were housed at room temperature, euthananized, and brains processed for immunohistochemistry (see below) or protein isolation (brain tissue dissection followed by snap-freezing in liquid nitrogen). An equivalent volume of saline was infused as a negative control.

**In vivo caspase activity assay.** Biotin-Val-Ala-Asp(OMe)-Fluoromethylketone (bVADfmk, MP Biomedicals) was used as an in vivo activate caspase molecular trap. 200nmoles of bVADfmk was diluted in 30µl sterile saline and infused by ICC prior to stroke. Brain tissue was harvested from rats or mice following treatment with bVADfmk and tMCAo, and was flash frozen on liquid nitrogen. Tissue was lysed by pestle disruption in cold CHAPS buffer containing protease inhibitors (Roche). For bVADfmk-caspase complex pulldown, protein lysates were pre-cleared by rocking with sepharose beads (GE Healthcare) for 1.0hr at 4°C. Pre-cleared lysate was centrifuged and the supernatant was transferred to 30µl of Streptavidin-agarose beads (Sigma) and rocked gently overnight at 4°C. Beads were washed/ centrifuged (300µl washes, 5000rpm for 5 minutes) 15 times with CHAPS buffer. After the final wash/pelleting, caspase-bVADfmk complexes were boiled off of streptavidin beads into 1x SDS sample buffer w/o reducing agent. Beads were pelleted at 14,000rpm for 10 minutes, and the supernatant was transferred to a fresh tube and resolved by SDS-PAGE. Saline was used as a vehicle control for bVADfmk.

**Intranasal Delivery of Pen1-XBIR3.** While under isofluorane anesthesia and lying on their backs, Pen1-XBIR3 (36.8 µM) was delivered to rats by administering 6µl drops to alternating nares every two minutes for 20 minutes (60µl total delivered). (Thorne, et al., Neuroscience 127 (2), 481-496 (2004)). Intranasal treatment was done prior to induction of stroke. Saline was used as a negative control. Brains were harvested for immunohistochemistry or western blotting.

**Immunohistochemistry (IHC), Cell Process Quantification, and Statistical Analysis.** Rats and mice were euthanized, perfused with heparin followed by fixation with 4% paraform-aldehyde. Sections were blocked for 1hr with 10% normal goat serum/1% BSA, incubated with primary antibody overnight at 4°C, washed with PBS-Triton-X100 (0.1%), incubated with the species appropriate Alexa Fluor-conjugated secondary antibody (Invitrogen) for 2hr at RT. Slides were also stained with Hoechst 33342 for 15min at RT (1µg/ml, Invitrogen) or with NeuroTrace fluorescent Nissl stain (1:300, Invitrogen) for 30min to stain for nuclei. Human samples were additionally treated with Sudan Black (1% in 70% EtOH) for 5min at RT and washed with 3 changes of PBS (3min each). For detection of fluorescent staining, sections were imaged with an upright Nikon fluorescent microscope using a SPOT digital camera and with a Perkin-Elmer Spinning Disc Confocal Imaging System. Quantification of neurons and axons was accomplished using the Cell Counter plug-in for ImageJ (NIH). For quantification in the rat brain, 20x magnification images were acquired from the dorsal motor cortex and the S1 somatosensory cortex forelimb region; both regions are contained are within the infarct penumbra (Fig. 1A). Single blind counts of processes or neurons were made in both regions of interest and then pooled for each individual animal. Three animals were used per cohort. For mouse brains, 20x magnification images were taken in the S 1 somato-sensory cortex forelimb region and similar counts were made as described below. Counts were made for NF-L/MAP-2 positive processes and NeuN positive cell bodies. Comparisons between groups used the student's t test, p-value: 0.05.

Human samples were also analyzed with DAB staining. Samples were incubated with 0.3% H2O2 for 30min, followed by blocking with 10% normal goat serum/1% BSA in PBS, and primary antibody incubation diluted in blocking buffer overnight at 4°C. After washing with PBS, slides were incubated with a species appropriate biotin-conjugated secondary antibody (Vector Laboratories) for 30min at RT. Samples were then incubated with ABC reagent (Vector Laboratories) for 30min and DAB stain for 10min. Samples were counterstained with hematoxylin and subsequently dehydrated with ethanol and cleared with 2 washes of xylene.

**Rat Hippocampal cultures.** Hippocampal neurons from E-18 rat embryos were dissected, dispersed in a defined serum free media, and plated on poly-D-lysine coated (0.1mg/ ml) tissue culture wells. The neurons were maintained in a serum free environment with Eagle's MEM and Ham's F12 (Gibco; Gaithersburg, MD) containing glucose (6mg/ml), insulin (25µg/ml), putrescine (60µM), progesterone (20nM), transferrin (100µg/ml), selenium (30nM), penicillin (0.5U/ml), and streptomycin (0.5µg/ml). Glial cells make up less than 2% of the culture. All cells were cultured for 8-10 days before treatment.

**Neuronal survival assay.** 4-hydroxynonenal (Cayman Chemicals) 3 µM as previously described was added to cultures in triplicate with and without Penl-XBIR3 (80nM). (Rabacchi, et al., Neurobiol Aging 25 (8), 1057-1066 (2004)). After 1 day of treatment cells number was quantified as previously described. (Rabacchi, et al., Neurobiol Aging 25 (8), 1057-1066 (2004)). Briefly, the cells were lysed in counting buffer and intact nuclei were counted using a hemocytometer. Nuclei of the healthy cells appear bright and have a clearly defined nuclear membrane while nuclei of dead cells disintegrate of appear irregularly shaped. Cell counts were performed in triplicate wells and averaged. % Survival is relative to control wells.

**Intranasal Penl-C6DN Prevents Cleavage of Caspase-6 Substrates.** Caspase-6 catalytic dominant negative (C6DN; C285A) was isolated and purified as described previously. Denault, J.B. and G.S. Salvesen, Expression, purification, and characterization of caspases. Curr Protoc Protein Sci, 2003. Chapter 21: p. Unit 21 13. Pen1 (Q-Biogene) was mixed at an equimolar ratio with purified C6DN and incubated overnight at 37°C to generate disulfide-linked Penl-C6DN. Linkage was assessed by 20% SDS-PAGE and Western blotting with anti-His and anti-Caspase-6 antibodies.

Male C57BL/6 mice (2-3 months old; >25g) were anesthetized using isoflurane (2%) delivered via an anesthesia mask. Penl-C6DN (30 µM) was delivered by administering 2 µl drops to alternating nares every minute for 10 min (20 µl total delivered). Thorne, R.G., et al., Delivery of insulin-like growth factor-I to the rat brain and spinal cord along olfactory and trigeminal pathways following intranasal administration. Neuroscience, 2004. 127(2): p. 481-96. Intranasal treatment was performed prior to 1hr transient Middle Cerebral Artery occlusion. Connolly, E.S., Jr., et al., Procedural and strain-related variables significantly affect outcome in a murine model of focal cerebral ischemia. Neurosurgery, 1996. 38(3): p. 523-31; discussion 532 and Komotar, R.J., et al., Neurologic assessment of somatosensory dysfunction following an experimental rodent model of cerebral ischemia. Nat Protoc, 2007. 2(10): p. 2345-7. Saline was used as a negative control. Brains were harvested for western blotting.

Microtubule-associated protein tau has been identified as molecular substrate of caspase-6. An antibody that binds to the neoepitope generated by caspase-6 cleavage of tau (anti-TauC3; Santa Cruz) was used to assay for caspase-6 inhibition by Pen1-C6DN during apoptosis in vivo. Anti-alpha-tubulin (Abcam) was used for a loading control.
**Amino Acid Sequence: c-IAP1 (Accession No. Q13490.2):** 
MHKTASQRLFPGPSYQNIKSIMEDSTILSDWTNSNKQKMKYDFSCELYRMST
YSTFPAGVPVSERSLARAGFYYTGVNDKVKCFCCGLMLDNWKLGDSPIQKH
KQLYPSCSFIQNLVSASLGSTSKNTSPMRNSFAHSLSPTLEHSSLFSGSYSSLSP
NPLNSRAVEDISSSRTNPYSYAMSTEEARFLTYHMWPLTFLSPSELARAGFYYI
GPGDRVACFACGGKLSNWEPKDDAMSEHRRHFPNCPFLENSLETLRFSISNLS
MQTHAARMRTFMYWPSSVPVQPEQLASAGFYYVGRNDDVKCFCCDGGLRC
WESGDDPWVEHAKWFPRCEFLIRMKGQEFVDEIQGRYPHLLEQLLSTSDTTG
EENADPPIIHFGPGESSSEDAVMMNTPVVKSALEMGFNRDLVKQTVQSKILTT
GENYKTVNDIVSALLNAEDEKREEEKEKQAEEMASDDLSLIRKNRMALFQQL
TCVLPILDNLLKANVINKQEHDIIKQKTQIPLQARELIDTILVKGNAAANIFKNC
LKEIDSTLYKNLFVDKNMKYIPTEDVSGLSLEEQLRRLQEERTCKVCMDKEV
SVVFIPCGHLVVCQECAPSLRKCPICRGIIKGTVRTFLS
**Amino Acid Sequence: c-IAP2 (Accession No. Q13489.2):** 
MNIVENSIFLSNLMKSANTFELKYDLSCELYRMSTYSTFPAGVPVSERSLARA
GFYYTGVNDKVKCFCCGLMLDNWKRGDSPTEKHKKLYPSCRFVQSLNSVNN
LEATSQPTFPSSVTNSTHSLLPGTENSGYFRGSYSNSPSNPVNSRANQDFSALM
RSSYHCAMNNENARLLTFQTWPLTFLSPTDLAKAGFYYIGPGDRVACFACGG
KLSNWEPKDNAMSEHLRHFPKCPFIENQLQDTSRYTVSNLSMQTHAARFKTF
 
FNWPSSVLVNPEQLASAGFYYVGNSDDVKCFCCDGGLRCWESGDDPWVQH
AKWFPRCEYLIRIKGQEFIRQVQASYPHLLEQLLSTSDSPGDENAESSIIHFEPG
EDHSEDAIMMNTPVINAAVEMGFSRSLVKQTVQRKILATGENYRLVNDLVLD
LLNAEDEIREEERERATEEKESNDLLLIRKNRMALFQHLTCVIPILDSLLTAGII
NEQEHDVIKQKTQTSLQARELIDTILVKGNIAATVFRNSLQEAEAVLYEHLFV
QQDIKYIPTEDVSDLPVEEQLRRLQEERTCKVCMDKEVSIVFIPCGHLVVCKD
CAPSLRKCPICRSTIKGTVRTFLS
**Amino Acid Sequence: XIAP (Accession No. P98170.2):** 
MTFNSFEGSKTCVPADINKEEEFVEEFNRLKTFANFPSGSPVSASTLARAGFLY
TGEGDTVRCFSCHAAVDRWQYGDSAVGRHRKVSPNCRFINGFYLENSATQS
TNSGIQNGQYKVENYLGSRDHFALDRPSETHADYLLRTGQVVDISDTIYPRNP
AMYSEEARLKSFQNWPDYAHLTPRELASAGLYYTGIGDQVQCFCCGGKLKN
WEPCDRAWSEHRRHFPNCFFVLGRNLNIRSESDAVSSDRNFPNSTNLPRNPSM
ADYEARIFTFGTWIYSVNKEQLARAGFYALGEGDKVKCFHCGGGLTDWKPS
EDPWEQHAKWYPGCKYLLEQKGQEYINNIHLTHSLEECLVRTTEKTPSLTRRI
DDTIFQNPMVQEAIRMGFSFKDIKKIMEEKIQISGSNYKSLEVLVADLVNAQK
DSMQDESSQTSLQKEISTEEQLRRLQEEKLCKICMDRNIAIVFVPCGHLVTCK
QCAEAVDKCPMCYTVITFKQKIFMS
**Amino Acid Sequence: NAIP (Accession No. Q13075.3):** 
MATQQKASDERISQFDHNLLPELSALLGLDAVQLAKELEEEEQKERAKMQK
GYNSQMRSEAKRLKTFVTYEPYSSWIPQEMAAAGFYFTGVKSGIQCFCCSLIL
FGAGLTRLPIEDHKRFHPDCGFLLNKDVGNIAKYDIRVKNLKSRLRGGKMRY
QEEEARLASFRNWPFYVQGISPCVLSEAGFVFTGKQDTVQCFSCGGCLGNWE
EGDDPWKEHAKWFPKCEFLRSKKSSEEITQYIQSYKGFVDITGEHFVNSWVQ
RELPMASAYCNDSIFAYEELRLDSFKDWPRESAVGVAALAKAGLFYTGIKDI
VQCFSCGGCLEKWQEGDDPLDDHTRCFPNCPFLQNMKSSAEVTPDLQSRGEL
CELLETTSESNLEDSIAVGPIVPEMAQGEAQWFQEAKNLNEQLRAAYTSASFR
HMSLLDISSDLATDHLLGCDLSIASKHISKPVQEPLVLPEVFGNLNSVMCVEG
EAGSGKTVLLKKIAFLWASGCCPLLNRFQLVFYLSLSSTRPDEGLASIICDQLL
EKEGSVTEMCVRNIIQQLKNQVLFLLDDYKEICSIPQVIGKLIQKNHLSRTCLLI
AVRTNRARDIRRYLETILEIKAFPFYNTVCILRKLFSHNMTRLRKFMVYFGKN
 
QSLQKIQKTPLFVAAICAHWFQYPFDPSFDDVAVFKSYMERLSLRNKATAEIL
KATVSSCGELALKGFFSCCFEFNDDDLAEAGVDEDEDLTMCLMSKFTAQRLR
PFYRFLSPAFQEFLAGMRLIELLDSDRQEHQDLGLYHLKQINSPMMTVSAYN
NFLNYVSSLPSTKAGPKIVSHLLHLVDNKESLENISENDDYLKHQPEISLQMQL
LRGLWQICPQAYFSMVSEHLLVLALKTAYQSNTVAACSPFVLQFLQGRTLTL
GALNLQYFFDHPESLSLLRSIHFPIRGNKTSPRAHFSVLETCFDKSQVPTIDQDY
ASAFEPMNEWERNLAEKEDNVKSYMDMQRRASPDLSTGYWKLSPKQYKIPC
LEVDVNDIDVVGQDMLEILMTVFSASQRIELHLNHSRGFIESIRPALELSKASV
TKCSISKLELSAAEQELLLTLPSLESLEVSGTIQSQDQIFPNLDKFLCLKELSVD
LEGNINVFSVIPEEFPNFHHMEKLLIQISAEYDPSKLVKLIQNSPNLHVFHLKCN
FFSDFGSLMTMLVSCKKLTEIKFSDSFFQAVPFVASLPNFISLKILNLEGQQFPD
EETSEKFAYILGSLSNLEELILPTGDGIYRVAKLIIQQCQQLHCLRVLSFFKTLN
DDSVVEIAKVAISGGFQKLENLKLSINHKITEEGYRNFFQALDNMPNLQELDIS
RHFTECIKAQATTVKSLSQCVLRLPRLIRLNMLSWLLDADDIALLNVMKERHP
QSKYLTILQKWILPFSPIIQK
**Amino Acid Sequence: survivin (Accession No. O15392.2):** 
MGAPTLPPAWQPFLKDHRISTFKNWPFLEGCACTPERMAEAGFIHCPTENEPD
LAQCFFCFKELEGWEPDDDPIEEHKKHSSGCAFLSVKKQFEELTLGEFLKLDR
ERAKNKIAKETNNKKKEFEETAEKVRRAIEQLAAMD
**Amino Acid Sequence: BRUCE (Accession No. Q9H8B7):**

## Claims

1. A compound for use in the treatment of ischemic injury in the central nervous system,
wherein the compound comprises a caspase inhibitor covalently conjugated to a cell-penetrating peptide and wherein the compound is administered intranasally,
wherein said caspase inhibitor is selected from the group consisting of:
(i) a caspase-6 dominant negative (C6DN), (ii) XBIR3, and (iii) a peptide that is capable of apoptotic target inhibition and has at least 70% homology with either a C6DN or a XBIR3; and
said cell-penetrating peptide is selected from the group consisting of penetratin-1, transportan, pIS1, Tat(48-60), pVEC, MAP, and MTS.

2. A compound for use according to claim 1 wherein the cell-penetrating peptide is penetratin-1.

3. A compound for use according to claim 1 wherein the peptide caspase inhibitor is C6DN.

4. A compound for use according to claim 1 wherein the peptide caspase inhibitor is XBIR3.

5. A compound for use according to claim 1 wherein the caspase inhibitor is administered during a window of time in which the inhibitor target is either expressed or active.

6. A compound for use according to claim 5 wherein the caspase inhibitor is XBIR3 and the administration occurs between the onset of the ischemic injury and 24 hours post reperfusion.

7. A compound for use according to claim 5 wherein the caspase inhibitor is Penl-C6DN and the administration occurs between 12 and 24 hours post reperfusion.

8. A compound for use according to 1 wherein the peptide caspase inhibitor is C6DN and the cell penetrating peptide is penetratin-1.

9. A compound for use according to claim 1 wherein caspase inhibitor is selected from the group consisting of (i) a disulfide-linked Penetratin1-C6DN, and (ii) a disulfide-linked Penetratin1 -XBIR3.

## Patentansprüche

1. Verbindung zur Verwendung bei der Behandlung einer ischämischen Verletzung des Zentralnervensystems,
wobei die Verbindung einen Caspase-Hemmer umfasst, der kovalent mit einem zellpenetrierenden Peptid konjugiert ist, und wobei die Verbindung intranasal verabreicht wird,
wobei der Caspase-Hemmer aus der Gruppe ausgewählt wird, die aus Folgendem besteht:
(i) eine dominant negative Caspase-6 (C6DN), (ii) XBIR3, und (iii) ein Peptid, das zu einer apoptotischen Zielhemmung imstande ist, und mindestens 70% Homologie zu entweder einer C6DN oder XBIR3 aufweist; und
das zellpenetrierende Peptid aus der Gruppe ausgewählt ist, die aus Penetratin-1, Transportan, pISI, Tat(48-60), pVEC, MAP und MTS besteht.

2. Verbindung zur Verwendung nach Anspruch 1, wobei das zellpenetrierende Peptid Penetratin-1 ist.

3. Verbindung zur Verwendung nach Anspruch 1, wobei der Peptid-Caspase-Hemmer C6DN ist.

4. Verbindung zur Verwendung nach Anspruch 1, wobei der Peptid-Caspase-Hemmer XBIR3 ist.

5. Verbindung zur Verwendung nach Anspruch 1, wobei der Peptid-Caspase-Hemmer während eines Zeitfensters verabreicht wird, in dem das Hemmer-Ziel entweder exprimiert oder aktiv ist.

6. Verbindung zur Verwendung nach Anspruch 5, wobei der Caspase-Hemmer XBIR3 ist, und die Verabreichung zwischen einem Auftreten der ischämischen Verletzung und 24 Stunden nach Reperfusion stattfindet.

7. Verbindung zur Verwendung nach Anspruch 5, wobei der Caspase-Hemmer Penl-C6DN ist und die Verabreichung zwischen 12 und 24 Stunden nach Reperfusion stattfindet.

8. Verbindung zur Verwendung nach Anspruch 1, wobei der Peptid-Caspase-Hemmer C6DN ist und das zellpenetrierende Peptid Penetratin-1 ist.

9. Verbindung zur Verwendung nach Anspruch 1, wobei der Caspase-Hemmer aus der Gruppe ausgewählt wird, die aus (i) einem Disulfid-gebundenen Penetratin1-C6DN und (ii) einem Disulfid-gebundenen Penetratin1-XBIR3 besteht.

## Revendications

1. Composé pour utilisation lors du traitement d'une lésion ischémique dans le système nerveux central,
dans lequel le composé comprend un inhibiteur de caspase conjugué de manière covalente à un peptide de pénétration cellulaire et dans lequel le composé est administré de manière intranasale,
dans lequel ledit inhibiteur de caspase est sélectionné à partir du groupe constitué de :
(i) un négatif dominant de caspase-6 (C6DN), (ii) XBIR3 et (iii) un peptide qui est capable d'une inhibition cible apoptotique et présente au moins 70 % d'homologie avec un C6DN ou un XBIR3 ; et
ledit peptide de pénétration cellulaire est sélectionné à partir du groupe constitué de pénétratine-1, de transportane, de pISI, de Tat(48-60), de pVEC, de MAP et de MTS.

2. Composé pour utilisation selon la revendication 1 dans lequel le peptide de pénétration cellulaire est la pénétratine-1.

3. Composé pour utilisation selon la revendication 1 dans lequel l'inhibiteur de caspase peptidique est le C6DN.

4. Composé pour utilisation selon la revendication 1 dans lequel l'inhibiteur de caspase peptidique est le XBIR3.

5. Composé pour utilisation selon la revendication 1 dans lequel l'inhibiteur de caspase est administré durant une fenêtre de temps dans laquelle la cible d'inhibiteur est soit exprimée soit active.

6. Composé pour utilisation selon la revendication 5 dans lequel l'inhibiteur de caspase est le XBIR3 et l'administration se produit entre le début de la lésion ischémique et 24 heures après la reperfusion.

7. Composé pour utilisation selon la revendication 5 dans lequel l'inhibiteur de caspase est le Pen1-C6DN et l'administration se produit entre 12 et 24 heures après la reperfusion.

8. Composé pour utilisation selon la revendication 1 dans lequel l'inhibiteur de caspase peptidique est le C6DN et le peptide de pénétration cellulaire est la pénétratine-1.

9. Composé pour utilisation selon la revendication 1 dans lequel l'inhibiteur de caspase est sélectionné à partir du groupe constitué de (i) Pénétratinel-C6DN liée à un disulfure et (ii) Pénétratine1-XBIR3 liée à un disulfure.
